# EUROPEAN PATENT APPLICATION

(11) **EP 4 029 445 A1**
(43) Date of publication of application: **20.07.2022**
(21) Application number: 21203555.4
(22) Date of filing: 25.07.2017
(51) Int. Cl.: A61B 5/15, A61B 5/151

(54) **DEVICE FOR RECEIVING BODILY FLUID FROM A SUBJECT**

(30) Priority: 29.07.2016 US 201662368440 P
(62) Divisional of application: 17751516.0
(71) Applicant: YourBio Health, Inc., Medford, MA 02155 (US)
(72) Inventor: BARONE, Vincent James, Niantic, 06357 (US); BRIGHTBILL, Jerry, Newton, 02349 (US); BUNNER, Bernard, Newton, 02464 (US); CHU, Li Yang, Medford, 02155 (US); GONG, Ping, Belmont, 02478 (US); JAMES, Scott, Epping, 03042 (US); SINGH, Inderpal, Norwood, 02062 (US)
(74) Representative: Potter Clarkson

(57) **Abstract**

The present invention generally relates to receiving bodily fluid through a device opening. In one aspect, the device includes a flow activator arranged to cause fluid to be released from a subject. A deployment actuator may actuate the flow activator in a deployment direction, which may in tum cause fluid release from a subject. The flow activator may also be moved in a retraction direction by a retraction actuator. In one aspect, the device may include a rotatable element that actuates the deployment actuator.

## Description

### RELATED APPLICATIONS

This Application claims the benefit under 35 US.C. § 119(e) of U.S. Provisional Application No. 62/368,440 filed on July 29, 2016. This application is incorporated by reference herein in its entirety.

### FIELD

The present invention generally relates to systems and methods for delivering to and/or receiving fluids or other materials, such as blood or interstitial fluid, from subjects, e.g., to or from the skin and/or beneath the skin.

### BACKGROUND

Phlebotomy or venipuncture is the process of obtaining intravenous access for the purpose of intravenous therapy or obtaining a sample of venous blood. This process is typically practiced by medical practitioners, including paramedics, phlebotomists, doctors, nurses, and the like. Substantial equipment is needed to obtain blood from a subject, including the use of evacuated (vacuum) tubes, e.g., such as the Vacutainer^{™} (Becton, Dickinson and company) and Vacuette^{™} (Greiner Bio-One GmBH) systems. Other equipment includes hypodermic needles, syringes, and the like. However, such procedures are complicated and require sophisticated training of practitioners, and often cannot be done in non-medical settings. Accordingly, improvements in methods of obtaining blood or other fluids from or through the skin are still needed.

### SUMMARY

In some embodiments, the present invention generally relates to devices and methods for receiving fluids from a subject, such as the reception and separation of blood to form plasma or serum. The subject matter of the present invention involves, in some cases, interrelated products, alternative solutions to a particular problem, and/or a plurality of different uses of one or more systems and/or articles.

In one aspect of the invention, a device for receiving fluid from a subject includes a housing including an opening to receive fluid into the housing, a device actuator and one or more needles that are arranged to cause fluid to be released from the subject. The device also includes a deployment actuator that moves the one or more needles in a deployment direction toward a subject and a rotatable element that contacts the deployment actuator to actuate the deployment actuator after actuation of the device actuator. The rotatable element is rotatable relative to the housing. In addition, actuation of the device actuator causes the rotatable element to rotate and move toward the deployment actuator.

In another aspect, a device for receiving fluid from a subject includes a housing comprising a base, a deployment actuator, and a carrier coupled to the deployment actuator. The device also includes a deployment actuator initiating element that actuates the deployment actuator by contacting and exerting a force on the deployment actuator. The initiating element has at least one spring member and the initiating element is positioned on the carrier. The device also includes a retraction actuator positioned between the carrier and the base such that movement of the carrier towards the base causes the retraction actuator to compress. A stiffness of the at least one spring member is greater than a stiffness of the retraction actuator.

In yet another aspect, a device for receiving fluid from a subject includes a housing comprising a base, where the base includes a hole and a device opening, and the hole is spaced from the device opening. The device also includes a deployment actuator and a flow activator comprising a plurality of needles. The device also includes an intermediate component coupling the flow activator to the deployment actuator, where the intermediate component extends across the hole and is movable through the hole. When the intermediate component is in the retracted position, the intermediate component seals the hole such that fluid communication through the hole is closed.

In yet another aspect, a device for receiving fluid from a subject includes a housing comprising a base, a deployment actuator that moves the one or more needles in a deployment direction toward a subject, and a carrier coupled to the deployment actuator. Upon actuation, the carrier moves toward the device opening prior to actuation of the deployment actuator.

In yet another aspect, a device for receiving fluid from a subject includes a housing comprising a base, a deployment actuator, and a carrier coupled to the deployment actuator. The device includes a deployment actuator initiating element that actuates the deployment actuator by contacting and exerting a force on the deployment actuator. The initiating element has at least one spring member and an initiating element positioned on the carrier. The at least one spring member contacts the carrier prior to the initiating element contacting the deployment actuator.

In yet another aspect, a device for receiving fluid from a subject includes a housing including an opening to receive fluid into the housing, where the housing also includes a base. The device also includes device actuator and one or more needles being arranged to cause fluid to be released from the subject. An anticoagulant coating is contained within the device and a covering element is positioned on the anticoagulant coating. The covering element has spaces that expose the anticoagulant coating.

In another aspect, the present invention encompasses methods of making one or more of the embodiments described herein, for example, a device for receiving fluid. In still another aspect, the present invention encompasses methods of using one or more of the embodiments described herein, for example, a device for receiving fluid.

Other advantages and novel features of the present invention will become apparent from the following detailed description of various non-limiting embodiments of the invention when considered in conjunction with the accompanying figures. In cases where the present specification and a document incorporated by reference include conflicting and/or inconsistent disclosure, the present specification shall control. If two or more documents incorporated by reference include conflicting and/or inconsistent disclosure with respect to each other, then the document having the later effective date shall control.

### BRIEF DESCRIPTION OF THE DRAWINGS

Non-limiting embodiments that incorporate one or more aspects of the invention will be described by way of example with reference to the accompanying figures, which are schematic and are not necessarily intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
Fig. 1 is a perspective view of a fluid receiving device in accordance with aspects of the invention;
Fig. 2 is an exploded view of the device shown in Fig. 1;
Fig. 3 is a sectional view of the device shown in Fig. 1;
Fig. 4 is a cross-sectional view of the device shown in Fig. 1;
Fig. 5 is an illustration of the fluid flow path into the device of Fig. 1, with the device shown in phantom;
Fig. 6A is a top down view of the device shown in Fig. 1;
Fig. 6B is a side view of the device shown in Fig. 1;
Fig. 7A is a bottom view of the device shown in Fig. 1;
Fig. 7B is a bottom view of the device shown in Fig. 7A, with a release liner hidden from view to reveal an adhesive beneath;
Fig. 7C is a bottom view of the device shown in Fig. 7B, with the adhesive hidden from view;
Fig. 8 is a perspective view of the device shown in Fig. 1, with the device cover shown in phantom to reveal a cap body beneath;
Fig. 9 is a perspective view of the device shown in Fig. 8, with the device cover hidden from view;
Fig. 10 is a perspective view of the device shown in Fig. 9, with the device actuator and cap body shown in phantom to reveal a rotary latch pusher, a rotary latch and carrier beneath;
Fig. 11 is a perspective view of the device shown in Fig. 10, with the device cover, device actuator and cap body hidden from view;
Fig. 12 is a perspective view of the device shown in Fig. 11, with the rotary latch pusher hidden from view;
Fig. 13 is a perspective view of the device shown in Fig. 12, with the rotary latch hidden from view;
Fig. 14A is a perspective view of the device shown in Fig. 13, with the carrier shown in phantom to reveal a deployment actuator beneath;
Fig. 14B is a perspective view of an underside of the carrier of Fig. 14A, depicting the deployment actuator coupled to the carrier;
Fig. 15 is a perspective view of the device shown in Fig. 14, with the carrier hidden from view;
Fig. 16 is a perspective view of the device shown in Fig. 15, with the deployment actuator hidden from view to reveal an opening in the base;
Fig. 17 is a bottom perspective view of the device shown in FIG. 1, with the release liner and adhesive hidden from view;
Fig. 18 is a cross-sectional view of a device in a ready state;
Fig. 19 is a cross-sectional view of the device shown in Fig. 18 undergoing a deployment sequence, with the rotary latch rotating off the ledges on the carrier;
Fig. 20 is a cross-sectional view of the device shown in Fig. 18 undergoing a deployment sequence, with the rotary latch starting to make contact with the deployment actuator;
Fig. 21 is a cross-sectional view of the device shown in Fig. 18, where the deployment actuator has been actuated and the flow activator is moved in the deployment direction;
Fig. 22 is a cross-sectional view of the device shown in Fig. 18, with the rotary latch is completely rotated and where the rotary latch pusher has fallen through a passage in the cap body;
Fig. 23 is a cross-sectional view of the device shown in Fig. 18 in the retracted orientation, with the flow activator retracted up from the device opening and the flow activator post sealing the opening in the base closed;
Fig. 24 is a perspective view of a rotary latch assembly in accordance with one aspect comprising a rotary latch, a rotary latch pusher and a carrier;
Fig. 25 is a perspective view of the assembly shown in Fig. 24, with the rotary latch shown in phantom to reveal carrier ledges underneath;
Fig. 26 is an enlarged view of the carrier ledges shown in Fig. 25, with the rotary latch and rotary latch pusher hidden from view;
Fig. 27A is a top perspective view of the rotary latch pusher shown in Fig. 24;
Fig. 27B is a side view of the rotary latch pusher shown in Fig. 24;
Fig. 27C is a top down view of the rotary latch pusher shown in Fig. 24;
Fig. 28 is a bottom perspective view of the rotary latch pusher shown in Fig. 24;
Fig. 29A is a top perspective view of the rotary latch shown in Fig. 24;
Fig. 29B is a bottom perspective view of the rotary latch shown in Fig. 24;
Fig. 29C is another bottom perspective view of the rotary latch shown in Fig. 24;
Fig. 30A is a top down view of a cap body according to one aspect;
Fig. 30B is a top perspective view of the cap body shown in Fig. 30A;
Fig. 30C is a bottom perspective view of the cap body shown in Fig. 30A, showing an interface;
Fig. 30D is another bottom perspective view of the cap body shown in Fig. 30A, showing the interface;
Fig. 30E is an enlarged perspective view of the interface at the underside of the cap body shown in Figs. 30C and 30D;
Fig. 30F is another enlarged perspective view of the interface at the underside of the cap body shown in Figs. 30C and 30D;
Fig. 30G is yet another enlarged perspective view of the interface at the underside of the cap body shown in Figs. 30C and 30D;
Fig. 31 is a perspective view of the bottom of the device in accordance with one aspect;
Fig. 32 is a perspective view of the bottom of the device shown in Fig. 31 with the release liner and adhesive hidden from view;
Fig. 33 is a perspective view of the bottom of the device shown in Fig. 32 with the base plate shown in phantom to reveal a covering element beneath;
Fig. 34 is a perspective view of the bottom of the device shown in Fig. 33 with the base plate hidden from view;
Fig. 35 is a perspective view of the bottom of the device shown in Fig. 34 with the covering element hidden from view to reveal a channel beneath;
Fig. 36 is an enlarged view of the device opening in accordance with one aspect, showing fingers from the covering element;
Fig. 37 shows the enlarged view of Fig. 36 with the base plate shown in phantom to reveal the covering element beneath;
Fig. 38 shows the enlarged view of Fig. 37 with the base plate hidden from view to reveal the covering element beneath;
Fig. 39A is a perspective view of the bottom of the device shown in Fig. 31 with the base plate lifted away from the device;
Fig. 39B shows the base plate of Fig. 40A flipped around to show the inside surface of the base plate;
Fig. 40 shows the enlarged view of Fig. 38 with the covering element hidden from view to reveal a channel beneath,
Fig. 41 shows a device held within a covering;
Fig. 42 is a flow chart depicting a manufacturing process in accordance with one aspect;
FIG. 43A is a top perspective view of a device within a covering and an interface cover in the form of a tray;
FIG. 43B is a bottom perspective view of the device, covering and interface cover shown in FIG. 43A;
FIG. 44 shows the device and interface cover of FIG. 43A spaced from the covering;
FIG. 45 shows an exploded view of the device, covering and interface cover of FIG. 43A; and
FIG. 46 shows a bottom perspective view of the exploded view shown in FIG. 45.

### DETAILED DESCRIPTION

Aspects of the invention are not limited in application to the details of construction and the arrangement of components set forth in the following description or illustrated in the drawings. For example, illustrative embodiments relating to piercing skin and receiving blood released from the pierced skin are discussed below, but aspects of the invention are not limited to use with devices that pierce skin and/or receive blood. Other embodiments may be employed, such as devices that receive other bodily fluids without piercing, and aspects of the inventions may be practiced or be carried out in various ways. Also, the phraseology and terminology used herein is for the purpose of description and should not be regarded as limiting.

According to one aspect, in some embodiments, the device has a rotary latch that rotates and moves downward in reaction to actuation of the device. Downward movement of the rotary latch causes the rotary latch to actuate a deployment actuator, which in turn moves the flow activator, such as one or more needles or microneedles, in a deployment direction to pierce a subject's skin. In one illustrative example, the deployment actuator is a snap dome or other bi-stable element that moves from a first stable state to a second stable state. The flow activator is coupled to the bi-stable element such that movement of the bi-stable element also moves the flow activator. Rotational movement of the rotary latch causes the rotary latch to clear an obstruction that previously prevented retraction of the flow activator. Upon clearing the obstruction, a retraction actuator is free to retract the flow activator. In one illustrative example, the retraction actuator is a spring that is initially compressed prior to actuation of the device. The spring is locked in compression due to an obstruction between the rotary latch and another component of the device. Upon rotation of the rotary latch, however, the rotary latch clears the obstruction and is free to move upward, allowing the spring to release its stored potential energy as it decompresses and moves in an upward retraction direction. This upward movement of the spring pushes a carrier holding the deployment actuator up with the spring, causing the flow activator, which is coupled to the deployment actuator, to retract as well.

According to one aspect, in some embodiments, the device has a rotary latch and a spring retraction actuator that are arranged in a force balance relative to one another during actuation of the device. In some embodiments, the rotary latch has one or more arms that act as springs having a higher collective stiffness (also referred to as its k-value, or spring constant) than that of the retraction spring. In some embodiments, the rotary latch sits on a carrier that holds the deployment actuator (e.g. snap dome or other bi-stable element). Actuation of the device actuator causes the rotary latch to rotate and move downward. As the rotary latch moves downward, force is exerted on both the retraction spring and the rotary latch arms. Because the retraction spring has a lower stiffness than the collective stiffness of the rotary latch arms, the retraction spring compresses, allowing the carrier, deployment actuator and flow activator to move toward the device opening until the bottom of the carrier contacts the base and the retraction spring cannot be compressed further. At this point, the rotary latch arms deflect, permitting the bottom of the rotary latch to contact the bi-stable element, which causes the bi-stable element to deflect downwards. This downward deflection of the bi-stable element accelerates the flow activator downward as well, driving the flow activator downward to pierce the skin of a subject. The stiffness relationship between the rotary latch and the spring retraction actuator ensures that the carrier has bottomed out and that the bi-stable element is in position for firing prior to actuation of the deployment actuator.

According to one aspect, in some embodiments, the base of the device has a hole through which a post moves, the post being coupled to the flow activator. Prior to actuation of the device, fluid communication through the hole is open such that fluid (liquid or gas) can pass through the hole and pressure is equilibrated on both sides of the hole. After retraction of the deployment actuator, however, the hole is sealed such that no fluid can pass through the hole. In some embodiments, the post includes a flange that contacts and seals against a sealing surface surrounding the hole. A sealing gasket may be included on the flange and/or the sealing surface to assist in creating an airtight seal.

According to one aspect, the device is arranged to pierce skin prior to subjecting the skin to vacuum. The inventors have recognized that, in some cases, subjecting skin to vacuum prior to piercing the skin may result in variable outcomes, as different types of skin respond differently when subjected to the same amount of vacuum. For example, loose skin may deform by a large amount when subjected to vacuum, while taut skin may deform a lesser amount when subjected to the same level of vacuum. The inventors have recognized that, in some embodiments, piercing skin prior to subjecting the skin to vacuum may result in more uniform fluid draws.

As a general overview, in some embodiments, the device is arranged to pierce the skin of a subject, subject the pierced skin to vacuum to draw fluid out of the skin, and collect the fluid inside the device. The device is arranged to deploy a plurality of microneedles into the skin. The device may be positioned on any suitable position of the subject, for example, on the arm or leg, on the back, on the abdomen, etc.

In some embodiments, the general sequence of events is as follows. A user actuates the device by, for example, pressing down on a button. Actuation of the device actuator causes an intermediate component to move downward and contact a deployment actuator. In some embodiments, the deployment actuator is a bi-stable element having two stable states, e.g. a snap dome. This contact "trips" the snap dome such that the snap dome moves in a deployment direction from a first stable state to a second stable state. In some embodiments, it is the central portion of the snap dome that moves from the first stable state to the second stable state. A flow activator, such as an array of microneedles, is coupled to the deployment actuator such that movement of the snap dome in the deployment direction also causes the microneedles to move in a deployment direction. This movement of the microneedles allows the microneedles to reach and pierce a subject's skin, causing release of bodily fluid such as blood. The bodily fluid enters the device through a device opening. After the microneedles are moved in the deployment direction, they are moved in a retraction direction by a retraction actuator. During retraction, a flow controller blocking fluid communication between the device opening and a vacuum chamber in the device may be opened, allowing vacuum to reach the device opening. As a result, fluid from the subject moves from the device opening into a storage chamber within the device. In some embodiments, a hydrophobic stop membrane separating the vacuum chamber from the storage chamber prevents blood from entering the vacuum chamber. The retracted microneedles are spaced up away from the device opening to prevent further contact with the subject. Further, in some embodiments, the deployment mechanism is arranged to "lock out" after a single actuation, meaning that the device cannot be actuated again such that the microneedles cannot be actuated to pierce a subject a second time, making the device a single-use device.

In some embodiments, the device operation is entirely mechanical and does not require a power source (e.g. electrical, battery) or software electronics.

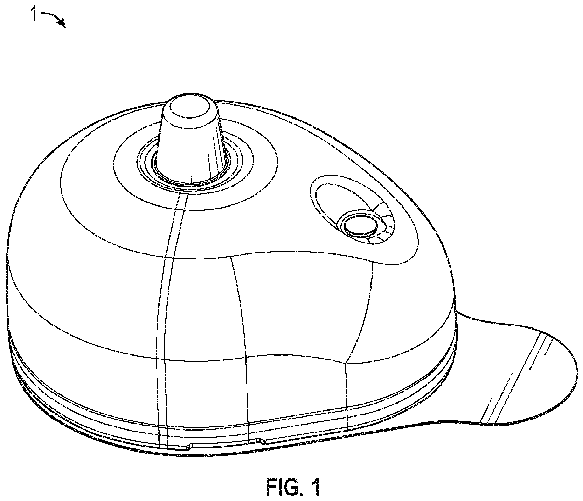

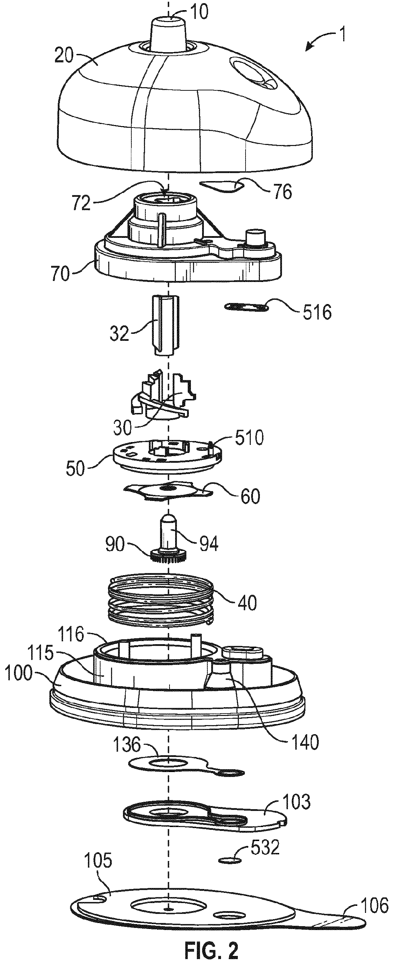

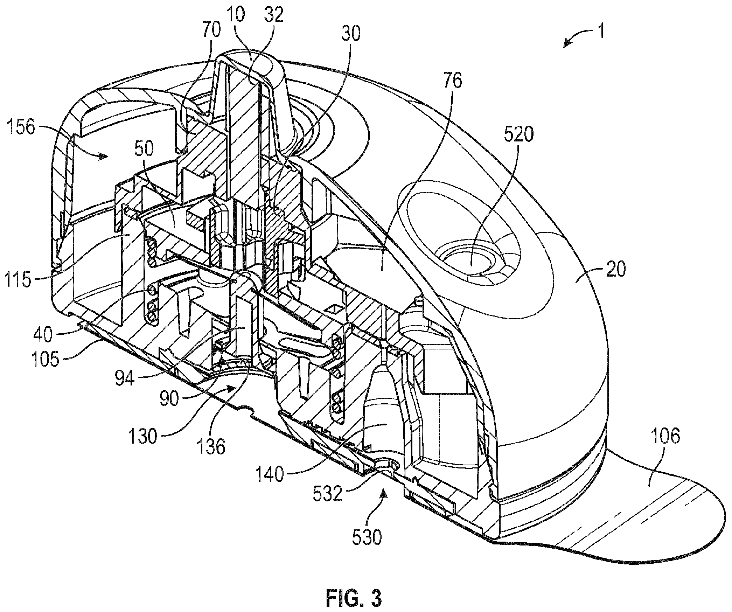

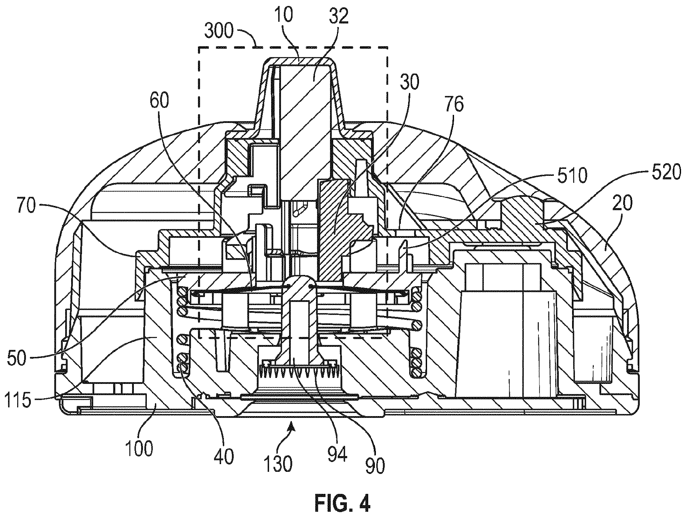

Turning to the figures, Fig. 1 is a perspective view depicting one embodiment of a device. The components of the device are shown in the exploded view of Fig. 2, the section view of Fig. 3 and the cross-sectional view of Fig. 4. The device 1 includes a device cover 20 and a base 100 that mate with one another to enclose various components within. An interface 105 such as a hydrogel adhesive, other adhesive, gasket, or other seal enhancing material may be included under the base to allow the device to hold the device in place during use. In some embodiments, the interface may aid in fluid collection by providing an airtight seal between the device and the skin that prevents escape of vacuum and/or escape of fluid.

In some embodiments, the device has a deployment mechanism that is made up of an assembly of components. As seen in Fig. 4, the deployment mechanism 300 includes a device actuator 10 such as a button, a rotary latch pusher 32, a rotary latch 30, a carrier 50 and a deployment actuator 60 such as a snap dome or other bi-stable element. The device actuator 10 is pressed down by the user to actuate the device. As will be described in further detail, downward movement of the device actuator 10 causes a rotary latch pusher 32 to move downward. As the rotary latch 30 moves downward, the bottom of the rotary latch contacts a bi-stable element 60 such as a snap dome, causing the bi-stable element to invert. The bi-stable element is coupled to a plurality of microneedles. In some embodiments, the bi-stable element is coupled to the microneedles via an intermediate post 94. Inversion of the bi-stable element from a raised up position to a deflected downward position causes the microneedles to move downward to pierce the subject's skin The inversion of the bi-stable element from the first stable state (raised up position) to the second stable state (deflected downward position) causes the microneedles to move in the deployment direction to pierce the subject's skin, which may initiate release of fluid from the subject.

Prior to actuation of the device, a retraction actuator, such as a spring 40, may be held in a configuration that stores potential energy. For example, the spring 40 is held in compression. Downward movement of the device actuator, rotary latch and carrier may further compress the spring. Rotation of the rotary latch 30 frees the spring 40 from being held in compression, and the spring relaxes to release potential energy, moving the carrier 50 upward as well. Upward movement of the carrier causes a piercing member on the carrier to pierce a seal 76, which opens fluid communication between the vacuum chamber and the device opening. Fluid from the subject is drawn through the device opening into the storage chamber 140.

Beneath the device cover 20, the device includes a cap body 70 that mates with or otherwise fits onto a raised portion 115 of the base 100. The interface between the cap body 70 and raised portion 115 of the base may be sealed so as to be airtight. In some embodiments, a sealing element such as a gasket 116 (seen in Fig. 2) may be used to create an airtight interface between the raised portion 115 and cap body 70.

A passage 72 for accommodating the rotary latch 30 and the rotary latch pusher 32 runs through the top of the cap body, leaving an opening at the top of the cap body 70. In some embodiments, the device actuator is attached to the top of the cap body such that this opening is sealed. In some cases, the device cover 20 has a hole through which the device actuator 10 extends. The device actuator may be attached to the device cover 20 such that the hole in the device cover 20 is sealed. As a result, the volume of space defined by the device cover 20, base 100 and cap body 70 is a sealed space that can hold a pre-packaged vacuum.

As can be seen in the section view of Fig. 3, prior to actuation of the device, the device can be divided into two sections: a section under pre-packaged vacuum and a section that is open to ambient pressure. The section under pre-packaged vacuum (also referred to as the vacuum chamber 156) is the volume bounded by the device cover 20, base 100 and cap body 70.

In some embodiments, what is referred to as the "vacuum chamber" is this sealed space defined by the device cover, base and cap body. Vacuum is pre-packaged into this space such that the air pressure in the vacuum chamber is lower than the ambient pressure outside the device. In some embodiments, during manufacture of the device, vacuum is introduced into the vacuum chamber by evacuating the chamber through a vacuum port, which, in some cases, may be located at the base.

The section that is open to ambient pressure is the space below the cap body 70 - the space occupied by the rotary latch 30, carrier 50, deployment actuator 60, retraction actuator 40, storage chamber 140, flow activator 90, device opening 130 and the channel 110 connecting the device opening to the storage chamber. Prior to actuation of the device, a flow controller such as seal 76 prevents fluid communication between the vacuum chamber 156 and the section of the device open to ambient pressure. In other words, prior to actuation of the device, the flow controller 76 prevents fluid communication between the vacuum chamber 156 and the device opening 130. In some embodiments, the flow controller is not at the device opening itself, but is downstream from the opening at a point along the pathway between the opening and the vacuum chamber.

After actuation of the device, during retraction of the carrier 50 and flow activator 90, the flow controller is opened, e.g. by piercing a seal, opening fluid communication from the vacuum chamber 156 to the storage chamber 140, the channel 110, and the device opening 130.

The base 100 includes the opening that receives fluid into the device and a storage chamber 140 that collects the fluid. The retraction spring 40 is sandwiched between the base 100 and the carrier 50. The bi-stable element 60 is held by the carrier and positioned on the bottom side of the carrier. In some embodiments, the snap dome is surrounded by one or more of the coils of the retraction spring 40. The top side of the carrier 50 may include one or more ledges that interact with features on the rotary latch 30 to properly position the rotary latch. The top side of the carrier may also include a piercing element that is used to pierce a vacuum seal as the carrier moves upward during retraction.

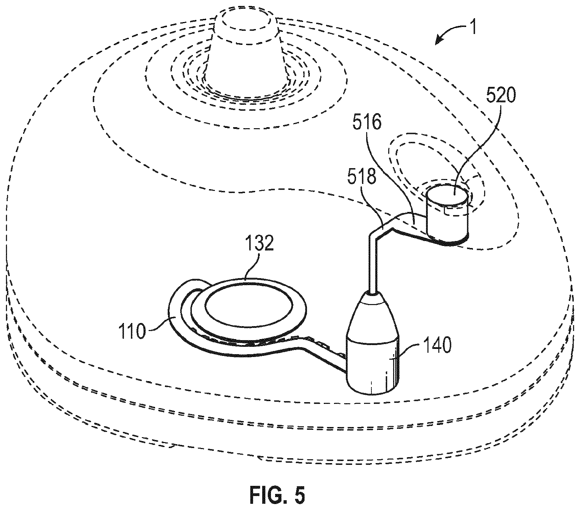

A schematic of the flow path of fluid into the device is shown in Fig. 5. Fluid enters the device opening via a capillary ring 132, which is connected to a storage chamber 140 via a channel 110. Another channel 518 then connects the storage channel 140 to an indicator 520. A fluidic stop membrane 516 prevents entry of fluid into the vacuum chamber. During the receiving of fluid, when fluid reaches the top of storage chamber 140, fluid may enter the channel 518 that connects storage chamber 140 to indicator 520. Fluid may enter and travel through channel 518 and arrive at the fluidic stop 516 due to capillary action, pressure differential, or via any other suitable force. Anticoagulant and/or other substances such as reagents may be coated along the fluid flow path.

According to one aspect, the device may enable an indication when the receiving of fluid is complete. Such indication may notify a user that the device can be removed from the skin. In the embodiment, shown in Fig. 5, a visual indication may be provided by the indicator 520. In some embodiments, the indicator is a light pipe or other refraction element that refracts light from the fluidic stop membrane 516. As a result, the color of any fluid on the fluidic stop membrane becomes visible on the indicator 520. As seen in Fig. 1, the indicator 520 is visible from outside the device, and thus the indicator indicates to a user whether or not fluid has filled the storage chamber 140.

In some embodiments, the indicator 520 may change color when the receiving of fluid is complete. In one example, the indicator 520 may change from clear to the color of the received fluid. The indicator 520 may include a flat disc of space that can receive and hold fluid. Indicator 520 may be in open communication with storage chamber 140. In some instances, indicator 520 may include a solid or liquid substance that changes color upon contact with the received fluid. In this way, a user may receive an indication that the receiving of fluid is complete without actual sight of the received fluid. For example, indicator 520 may turn a color that is different than the actual collected fluid. In some embodiments, the device may include an indicator cover that may be transparent or translucent to allow a user to view indicator 520. In some embodiments, indicator cover may be tinted a color to change the appearance of the color of the fluid. In some cases, indicator cover may be removable. Of course, it should be appreciated that the indication may be visual, audible, or tactile, as this aspect is not limited in this regard. For example, filling of storage chamber 140 may trigger the device to emit an audible sound indicating that the receiving of fluid is complete. In some instances, the audible sound may be a mechanical click due to interaction between the device actuator, release element, effector, retraction actuator, deployment actuator, and/or flow activator. In some instances, the audible sound may be an alarm that is triggered due to fluid reaching the top of the storage chamber 140. Alternatively or in addition, the user may receive tactile feedback indicating that the receiving of fluid is complete. For example, the device actuator, release element, effector, retraction actuator, deployment actuator, and/or flow activator may be arranged to interact such that the user actuating device actuator experiences a sudden increase or decrease in physical resistance from the device actuator. As another example, the components of the device may include a detent-type interaction that provides tactile feedback to the user. Furthermore, the indications, feedback, and/or alarms may occur at any point in the device actuation process, as indications are not limited to the completion of the receiving of fluid. For example, the device may enable an indication when vacuum has been released, when the flow activator has been deployed and/or retracted, when the receiving of fluid has begun, etc. The device may also enable an indication or alarm when an insufficient volume fluid has been received, or if the type of fluid received is inappropriate.

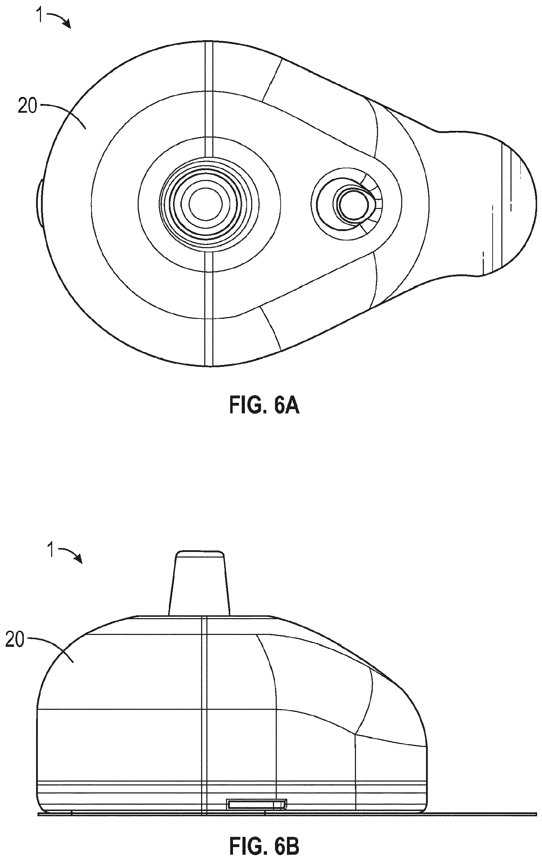

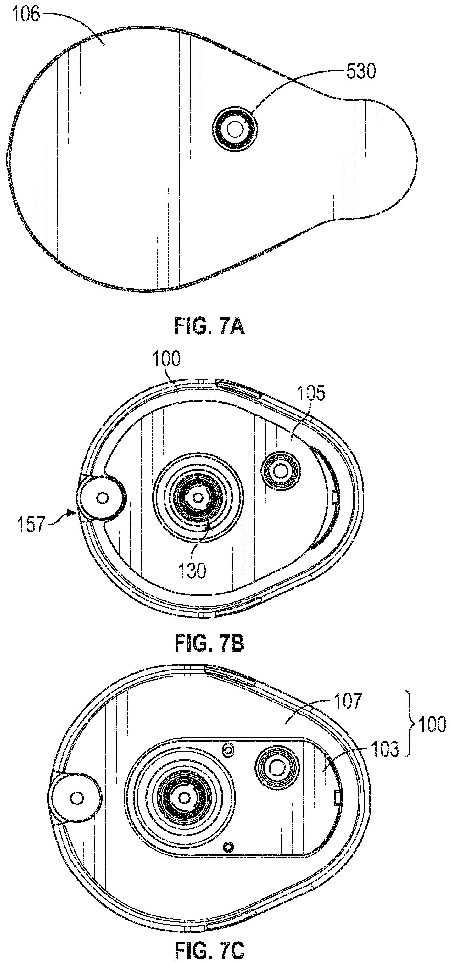

Each component of one illustrative embodiment the device will now be generally discussed. The housing of the device, which includes device cover 20, is seen in Fig. 6A, which is a top-down view of the device, and Fig. 6B, which is a side view of the device. The bottom side of the device is shown in Figs. 7A-7C. As seen in Fig. 7A, the bottom of the device includes an interface cover 106 such as a release liner that is removed by the user to reveal an interface 105 beneath, seen in Fig. 7B, which shows the bottom of the device without the release liner. The interface may have adhesive properties, and so the release liner 106 may be used to prevent the interface 105 from prematurely adhering to other objects. The base 100 and device opening 130 are also shown in Fig. 7B. In some embodiments, a vacuum fill port 157 may be located at the bottom of the device. This fill port is in fluid communication with the vacuum chamber inside the device. During manufacture of the device, air is evacuated from the vacuum chamber through the vacuum fill port 157 in order to pre-package the vacuum chamber with vacuum that is less than less than ambient pressure outside the device. The vacuum fill port 157 may then be covered by a seal 158 to contain the vacuum inside the vacuum chamber.

It should be appreciated that, in other embodiments, vacuum is not pre-packaged within the device. Instead, for example, vacuum may be generated during actuation of the device or vacuum from an alternative vacuum source may be used.

In some embodiments, as seen in Fig. 7C, the base 100 includes a base plate 103 that attaches to main base body 107. A covering element may be sandwiched between the base plate 103 and the main base body 107, as will be discussed in detail in a later section. The base, including the main base body and the base plate, may be made of polyester or any other suitable material.

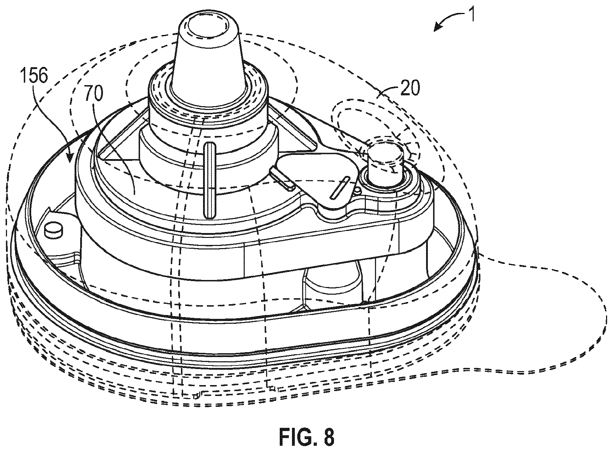

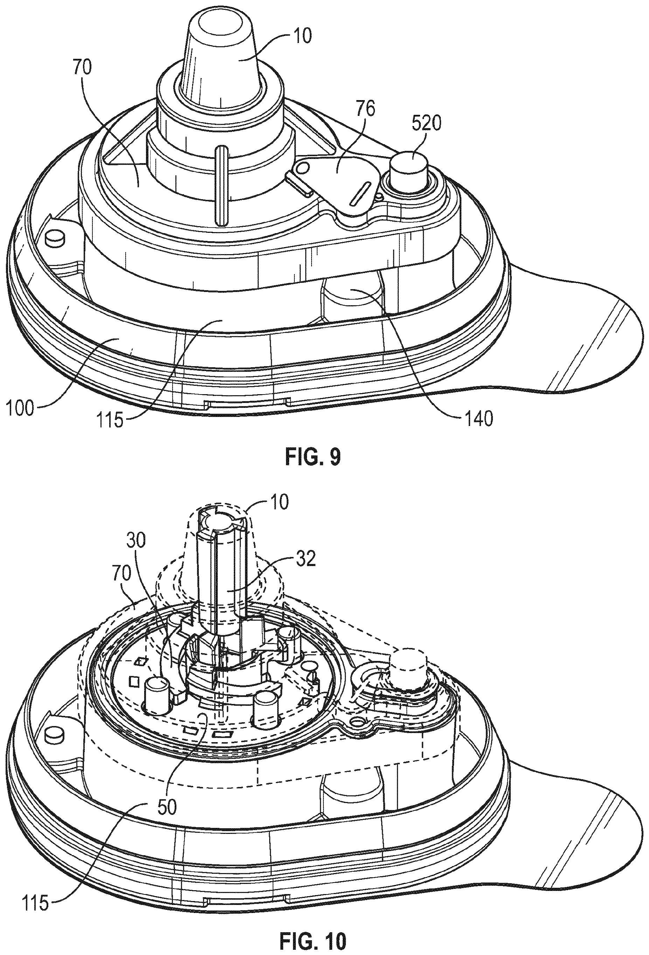

Fig. 8 is a perspective view of the device, with the device cover 20 shown in phantom to reveal a cap body 70 beneath. A vacuum chamber 156 is the volume of space bounded by the base 100, cap body 70 and device cover 20. As seen in Fig. 9, in which the device cover is hidden from view, the device actuator 10 attaches to the cap body 70, and the cap body attaches to a raised portion 115 of the base 100. A seal 76 is attached to a top surface of the cap body. The cap body 70 has a hole that, when exposed, fluidly connects the vacuum chamber to the storage chamber and the device opening. The seal 76 covers this hole to serve as a flow controller that closes fluid communication between the vacuum chamber and the device opening prior to device actuation. The cap body 70 is also connected to the indicator 520.

Looking to the components located under the device actuator 10 and cap body 70, Fig. 10 shows the device actuator 10 and cap body 70 in phantom to reveal a rotary latch pusher 32, rotary latch 30 and carrier 50. Prior to actuation, rotary latch pusher 32 is held in a position under device actuator 10 such that downward depression of the device actuator 10 causes an internal surface of the device actuator 10 to contact a top surface of the rotary latch pusher, pushing the rotary latch pusher downward in a deployment direction.

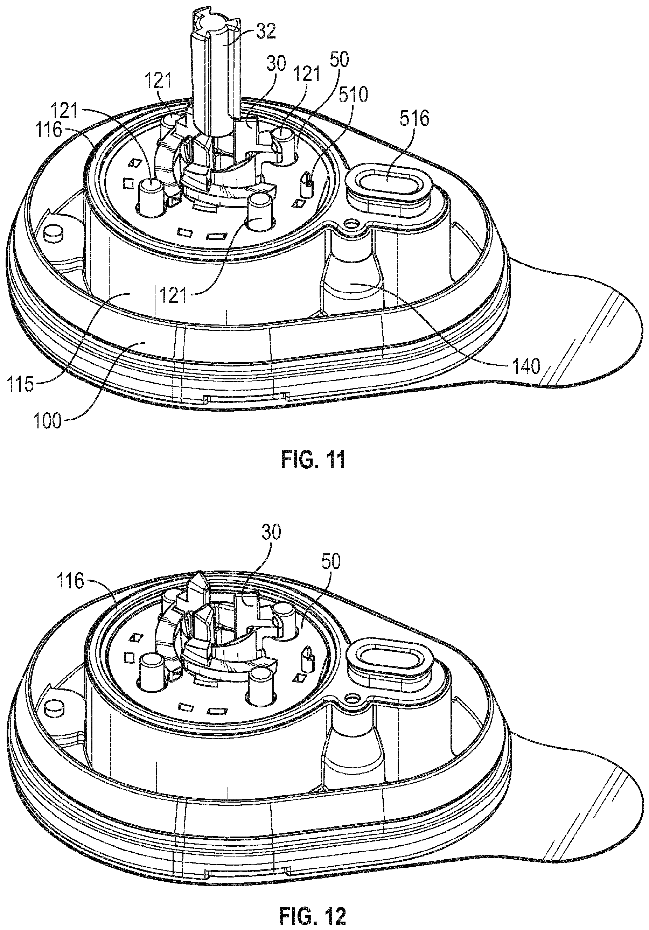

In Fig. 11, in which the device actuator and cap body are hidden from view, the assembly comprising the rotary latch pusher 32, rotary latch 30 and carrier 50 can be seen. Prior to actuation, the rotary latch pusher 32 rests upon the rotary latch 30, and the rotary latch 30 rests upon the carrier 50. In some embodiments, posts 121 from the base 100 extend through corresponding holes in the carrier. As the carrier 50 moves toward the base 100, the posts 121 extend further through the holes of the carrier. Conversely, as the carrier 50 moves away from the base 100, the posts 121 are pulled back such that they do not extend as far through the holes of the carrier. In some cases, having the posts 121 extend through the carrier may help to keep the carrier in a proper rotational orientation as the carrier moves relative to the base. This may help to ensure that the piercing element 510 is properly aligned with a vacuum chamber seal to pierce the seal during movement of the carrier in the retraction direction.

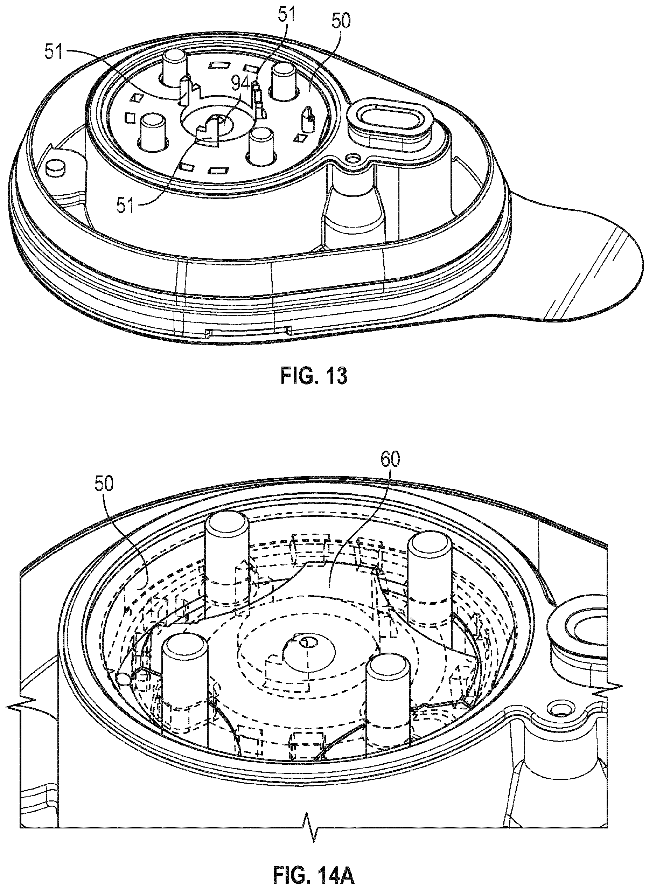

The rotary latch 30, which is positioned beneath the rotary latch pusher 32, can be more clearly seen in Fig. 12, in which the rotary latch pusher 32 is hidden from view. In Fig. 13, the rotary latch pusher 32 is hidden from view to reveal protrusions 51 on the carrier 50. In some embodiments, prior to actuation of the device, the rotary latch 30 rests upon these protrusions 51 to prevent the arms of the rotary latch 30 from contacting the top surface of the carrier 40. The interaction between the rotary latch pusher, rotary latch and carrier will be discussed in greater detail in a later section.

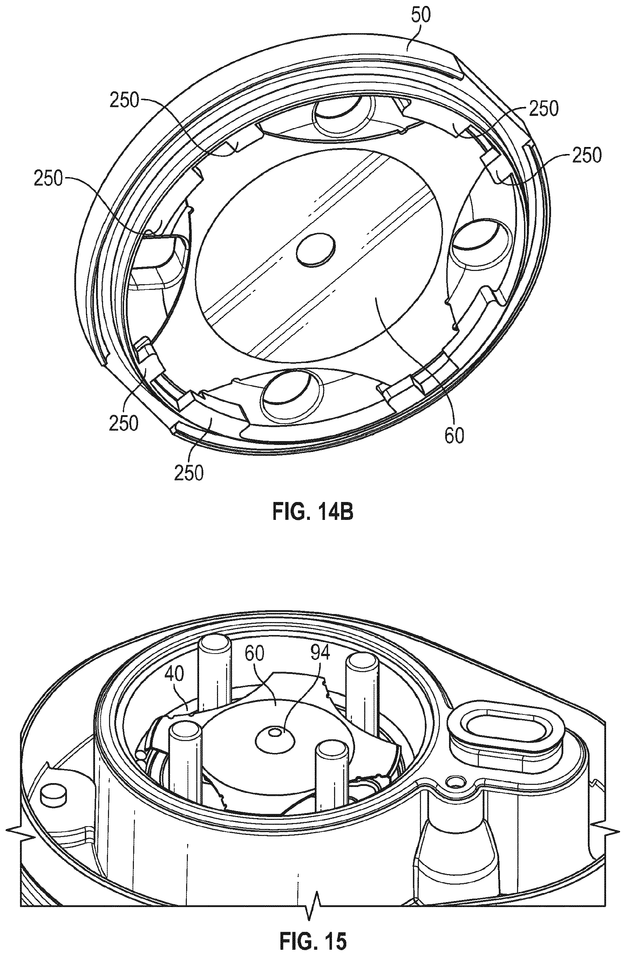

The deployment actuator 60 is located beneath the carrier 50, as shown in Fig. 14A, in which the carrier 50 is shown in phantom. The carrier 50 holds the deployment actuator 60 such that movement of the carrier 50 moves the deployment actuator 60 along with the carrier. The underside of the carrier 50 is shown in Fig. 14B. Tabs 250 on the underside of the carrier 50 hold the deployment actuator 60 to the carrier.

The position of the deployment actuator 60 relative to the base 100 prior to actuation of the device is seen in Fig. 15, in which the carrier is hidden from view. The top of the post 94 that couples the deployment actuator to the flow activator can also be seen in Fig. 15. In addition, the retraction actuator 40 lies beneath the carrier. The retraction actuator 40 is sandwiched between the base 100 and the carrier 50 such that, when the carrier 50 moves toward the base, the retraction actuator 40 is compressed. When the retraction actuator 40 is permitted to decompress, it pushes the carrier 50 (and with the carrier, the deployment actuator and flow activator), up away from the base in the retraction direction. In some embodiments, the retraction actuator is coupled to the bottom of the carrier. The retraction actuator may surround the deployment actuator, which is also coupled to the bottom of the carri er.

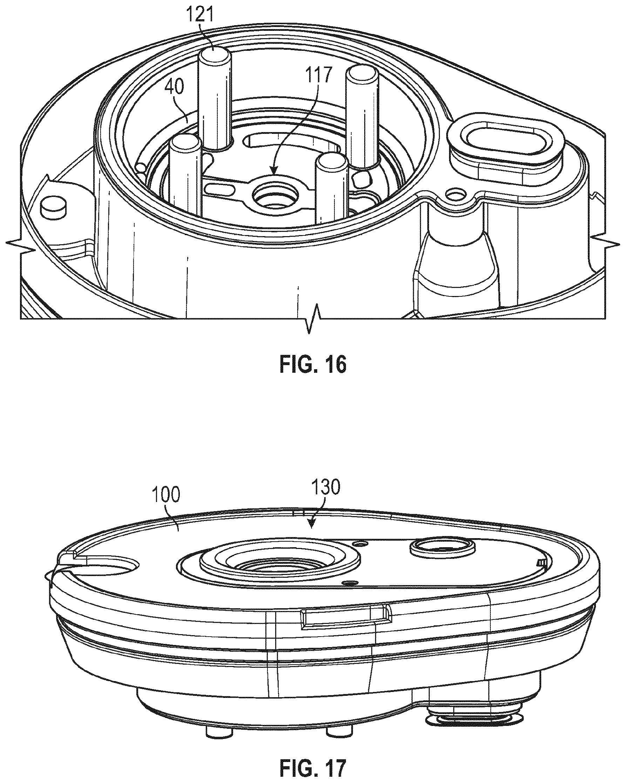

The portion of the base 100 beneath the deployment actuator can be seen in Fig. 16, in which the deployment actuator is hidden from view. As seen in Fig. 16, the base includes a hole 117 through which the post that couples the flow activator to the deployment actuator passes. This hole 117 is in fluid communication with the device opening 130 located on the bottom of the device, shown in Fig. 17, which depicts the underside of the device. In some embodiments, the hole 117 in the base is concentrically aligned with the device opening 130.

In some embodiments, the flow activator may include one or more needles. The needles may be arranged in a variety of different ways, depending on the intended application. For example, the needle(s) may have a length of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, less than about 10 micrometers, etc. The needle(s) may also have a largest cross-sectional dimension of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, less than about 10 micrometers, etc. For example, in one embodiment, the needle(s) may have a rectangular cross section having dimensions of 175 micrometers by 50 micrometers. In one set of embodiments, the needle(s) may have an aspect ratio of length to largest cross-sectional dimension of at least about 2:1, at least about 3:1, at least about 4:1, at least 5:1, at least about 7:1, at least about 10:1, at least about 15:1, at least about 20:1, at least about 25:1, at least about 30:1, etc.

In one embodiment, the needle(s) is(are) a microneedle(s). Typically, a microneedle will have an average cross-sectional dimension (e.g., diameter) of less than about a millimeter. It should be understood that references to "needle" or "microneedle" as discussed herein are by way of example and ease of presentation only, and that in other embodiments, more than one needle and/or microneedle may be present in any of the descriptions herein.

The microneedles may be hollow or solid, and may be formed from any suitable material, e.g., metals, ceramics, semiconductors, organics, polymers, and/or composites. Examples include, but are not limited to, medical grade stainless steel, titanium, nickel, iron, gold, tin, chromium, copper, alloys of these or other metals, silicon, silicon dioxide, and polymers, including polymers of hydroxy acids such as lactic acid and glycolic acid polylactide, polyglycolide, polylactide-co-glycolide, and copolymers with polyethylene glycol, polyanhydrides, polyorthoesters, polyurethanes, polybutyric acid, polyvaleric acid, polylactide-co-caprolactone, polycarbonate, polymethacrylic acid, polyethylenevinyl acetate, polytetrafluorethylene, polymethyl methacrylate, polyacrylic acid, or polyesters.

In some cases, more than one needle or microneedle may be used. For example, arrays of needles or microneedles may be used, and the needles or microneedles may be arranged in the array in any suitable configuration, e.g., periodic, random, etc. In some cases, the array may have 3 or more, 4 or more, 5 or more, 6 or more, 10 or more, 15 or more, 20 or more, 35 or more, 50 or more, 100 or more, or any other suitable number of needles or microneedles. Typically, a microneedle will have an average cross-sectional dimension (e.g., diameter) of less than about a micron.

In one illustrative embodiment, the flow activator includes an array of microneedles that are arranged in a 7.5 mm diameter circular pattern with 30 microneedles around the circumference. Each of the microneedles is 1 mm long and 0.350 mm wide.

According to one aspect, actuation of the device initiates a sequence of operations. In some embodiments, once the device is actuated (e.g. a user presses a button), the sequence of operations occurs at a predefined speed and order that are independent of how long the user remains pressing the device actuator.

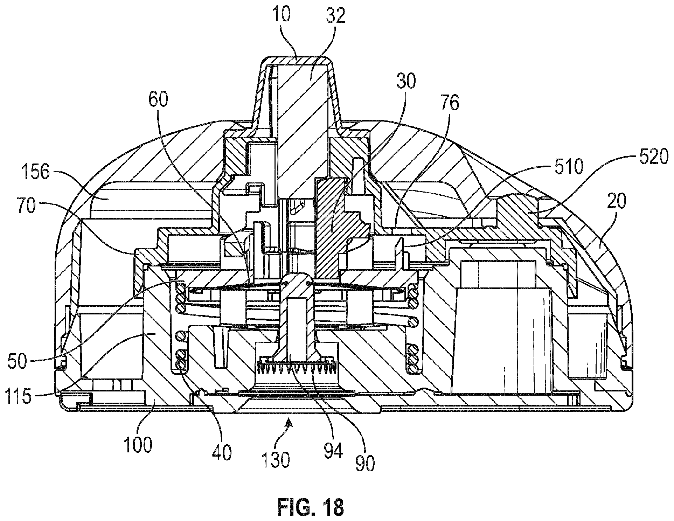

An illustrative embodiment of a sequence of operations will now be described. Figs. 18-23 depict the changes in a cross-section of the device as the device undergoes the sequence of operations that occur after device actuation.

Fig. 18 shows the device in the ready position prior to actuation. The rotary latch pusher 32 is positioned adjacent to the device actuator 10 and a portion of the pusher 32 is located within the passage of the cap body 70. The bottom of the pusher 32 is in contact with the rotary latch 30 and the rotary latch 30 rests upon the ledges of the carrier 50. The retraction actuator 40 is in a first compressed state between the carrier 50 and the base 100. The bottom of the carrier 50 is spaced from the base 100 and the flow activator 90 is spaced from the device opening 130 such that the flow activator cannot reach a subject's skin. Vacuum is stored within the vacuum chamber 156 and fluid communication between the vacuum chamber 156 and the device opening 130 is closed by the seal 76. The piercing element 510 on the carrier is spaced from the vacuum chamber seal 76.

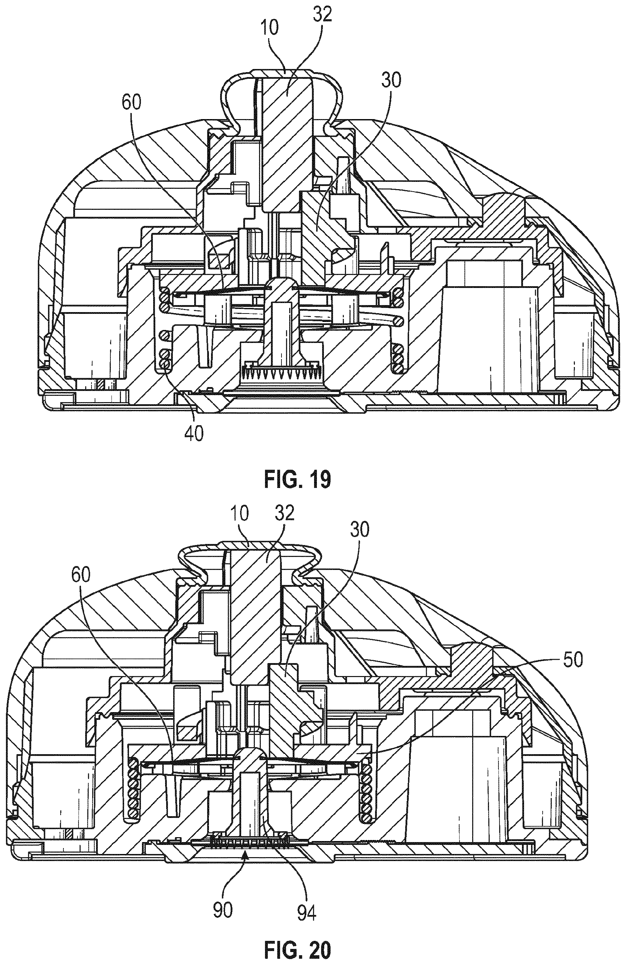

Fig. 19 shows the device at the start of actuation of the device. The device actuator 10 is compressed as a user presses down on the device actuator 10. The rotary latch pusher 32, which is in contact with the device actuator 10, starts to move downward in reaction to the device actuator 10 being pressed.

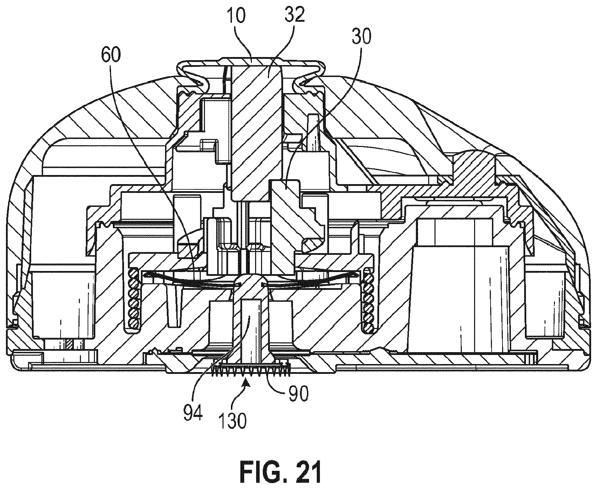

Next, as seen in Fig. 20, with the device actuator 10 fully depressed, the rotary latch pusher 32 has moved downward. Downward movement of the rotary latch pusher 32 causes the pusher 32 to push against the rotary latch 30, which causes the rotary latch 30 to rotate and move downward. Rotation of the rotary latch 30 causes the arms of the rotary latch to clear the ledges on the carrier 50 and allows the arms to contact the top carrier face directly. Downward movement of the rotary latch 30 pushes the carrier 50 downward as well, compressing the retraction actuator 40 until the bottom of the carrier 50 bottoms out against the inside surface of the base 100. The deployment actuator 60, post 94, and flow activator 90, which are coupled to the carrier, are also moved downward as the carrier 50 moves downward. The flow activator 90 is now within deployment distance of the subject's skin. The bottom of the rotary latch 30 is in contact with the top of the deployment actuator 60. As the rotary latch 30 presses on the deployment actuator 60, the deployment actuator 60 is actuated (e.g., the force exerted on the deployment actuator 60 is greater than the "trip" force of the deployment actuator, causing the deployment actuator to move from a first undeployed state to a second deployed state. In some embodiments, the deployment actuator is a bi-stable element that inverts from a first stable state to a second stable state when the "trip" force is exceeded). The result is shown in Fig. 21, which depicts the flow activator 90 in the fully deployed state for insertion into a subject's skin. A comparison between Fig. 20 and Fig. 21 shows the change in conformation of the deployment actuator 60 from a first undeployed state (Fig. 20) to a deployed state (Fig. 20). These two states may be bi-stable, i.e., force must be exerted on the deployment actuator to move from one state to the other and vice versa, or, in some embodiments, only one of the states is stable such that the deployment actuator is biased toward one of the states.

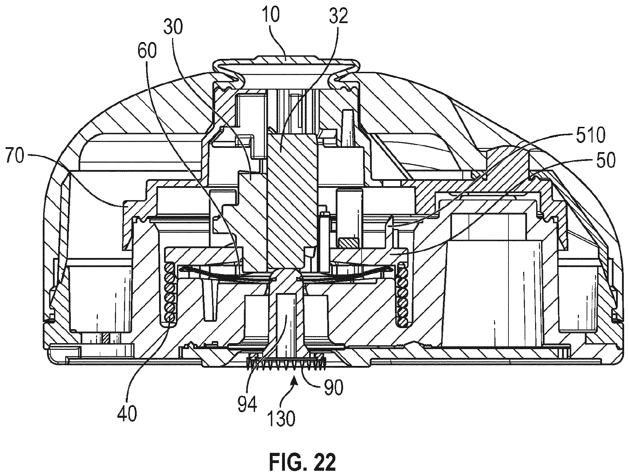

At this stage, the rotary latch 30 has rotated from its initial position relative to the cap body 70 and the pusher 32. As a result, as seen in Fig. 22, the pusher 32 falls through the passage in the cap body 70 and a passage within the rotary latch 30. In some embodiments, the pusher 32 drops until it comes into contact with the top of the post 94. In some embodiments, where the post does not extend through the deployment actuator 60, the pusher 32 would drop until it comes into contact with the deployment actuator 60. As will be discussed in detail in the next section, rotation of the rotary latch 30 relative to the cap body 70 allows the rotary latch 30 to clear indentations on the cap body and enter vertical grooves in the cap body, allowing the rotary latch 30 to move upward into the passage of the cap body 70. Contact between the top of the rotary latch and the indentations on the cap body 70 had previously prevented the rotary latch from moving upward. Because the rotary latch 30 has cleared these indentations, the retraction actuator 40 is permitted to relax from its compressed state, bringing the device to the retraction stage.

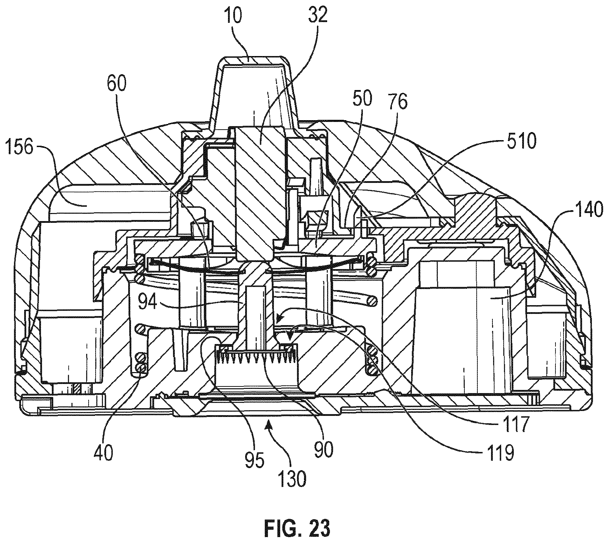

As seen in Fig. 23, the retraction actuator 40 has expanded from its decompressed state, pushing the carrier 50 upward such that the piercing element 510 has pierced through the vacuum seal 76 that previously closed fluid communication between the device opening and the vacuum chamber. With the seal 76 pierced open, the vacuum chamber 156 is in fluid communication with the storage chamber 140 and the device opening 130.

In the retracted state, the post 94 is at a height relative to the base 100 such that a flange 95 on the post 94 presses up against a sealing surface 119 surrounding the hole 117 at the base. The flange 95 is located below the hole 117, on the side of the hole that leads to the device opening 130. Contact between the flange 95 and the sealing surface 119 seals the hole 117 closed such that fluids cannot pass through the hole 117. This prevents vacuum from the vacuum chamber from passing through the hole 117, and also prevents entry of fluid from the subject from entering through the hole 117, ensuring that fluids enter the device via the channel between the device opening and the storage chamber.

Decompression of the retraction actuator 40 has also moves the pusher 32 and the rotary latch 30 up into the passage of the cap body 70. In the embodiment shown in Fig. 23, after retraction, the top of the pusher 32 remains spaced from the inner top surface of the device actuator 10, such that the device cannot be actuated again. Furthermore, the rotary latch 30 has rotated into a locked position within the cap body 70 to prevent further actuation of the device.

According to one aspect, the device includes a deployment mechanism that is made up of an assembly of components. The interaction between these components will now be described in detail.

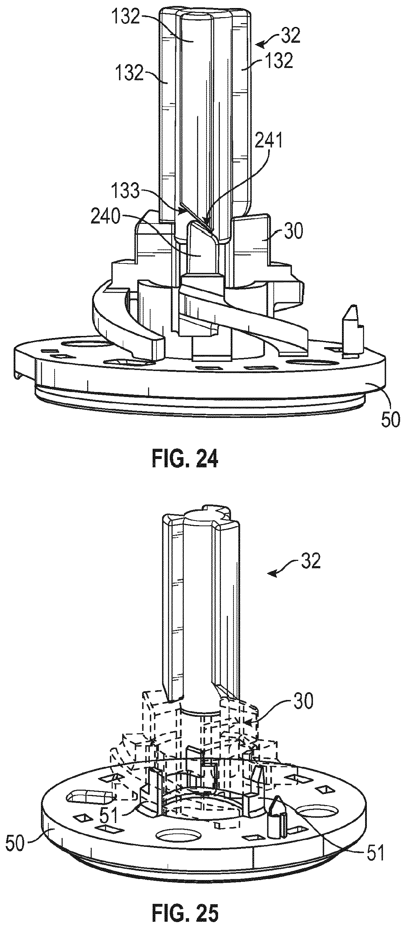

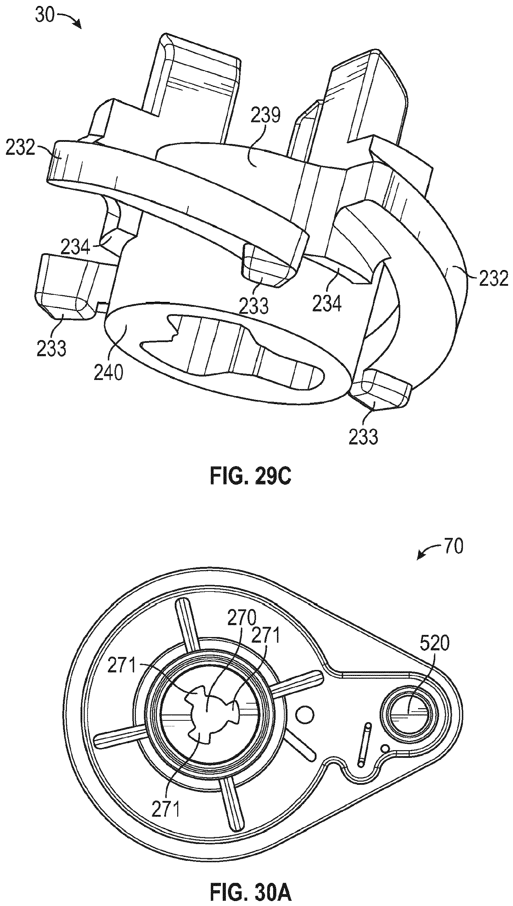

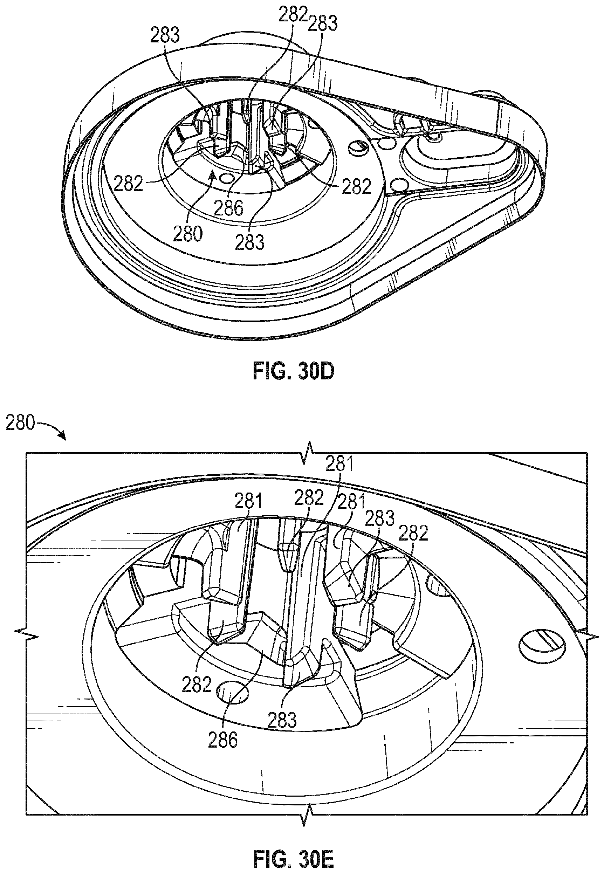

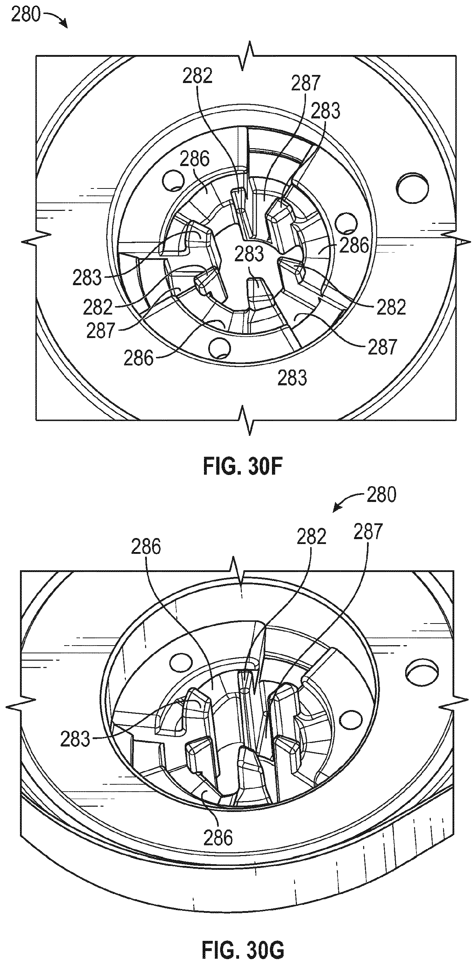

As discussed previously, the deployment mechanism includes a rotary latch pusher 32, a rotary latch 30, and a carrier 50 that holds the deployment actuator. These components are shown in detail in Figs. 24-29C. The rotary latch pusher 32 and rotary latch 30 also interact with a cap body 70, which is shown in detail in Figs. 30A-30G.

As seen in Fig. 24, prior to actuation of the device, the rotary latch pusher 32 sits upon the rotary latch 30, and the rotary latch 30 sits upon the carrier 50. The pusher 32 has a plurality of fins 131. The rotary latch 30 has a plurality of legs 240 that correspond with the number of fins on the pusher 32 such that each fin on the pusher is paired with a leg on the rotary latch.

The bottom of each fin 131 has a sloped surface 133 that is angled to correspond to a sloped surface 241 on a leg 240 of the rotary latch 30. Downward movement of the pusher 32 causes the slope 133 on the fin 131 of the pusher 32 to slide against the slope 241 on the leg 240 of the rotary latch. Because of the angle of the slopes 133, 241, sliding contact between the two surfaces causes the rotary latch 30 to rotate. In this embodiment, the rotary latch 30 rotates clockwise when viewed from above (e.g., to the left in Fig. 24).

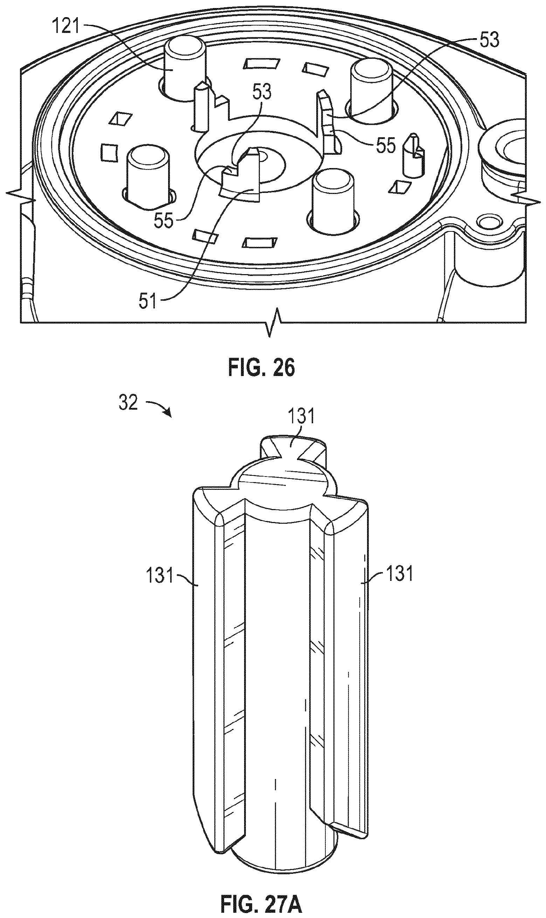

The rotary latch 30 sits upon the carrier 50. In some embodiments, prior to actuation, the rotary latch 30 sits upon protrusions 51 on the top surface of the carrier 50. In Fig. 25, the rotary latch 30 is shown in phantom to reveal the protrusions 51 beneath. The protrusions 51 are best seen in Fig. 26, in which the rotary latch 30 is hidden from view. Each protrusion 51 has a ledge surface 55 and a side surface 53. The rotary latch 30 initially rests upon the ledge surfaces 55 of the carrier protrusions 51, as will be discussed.

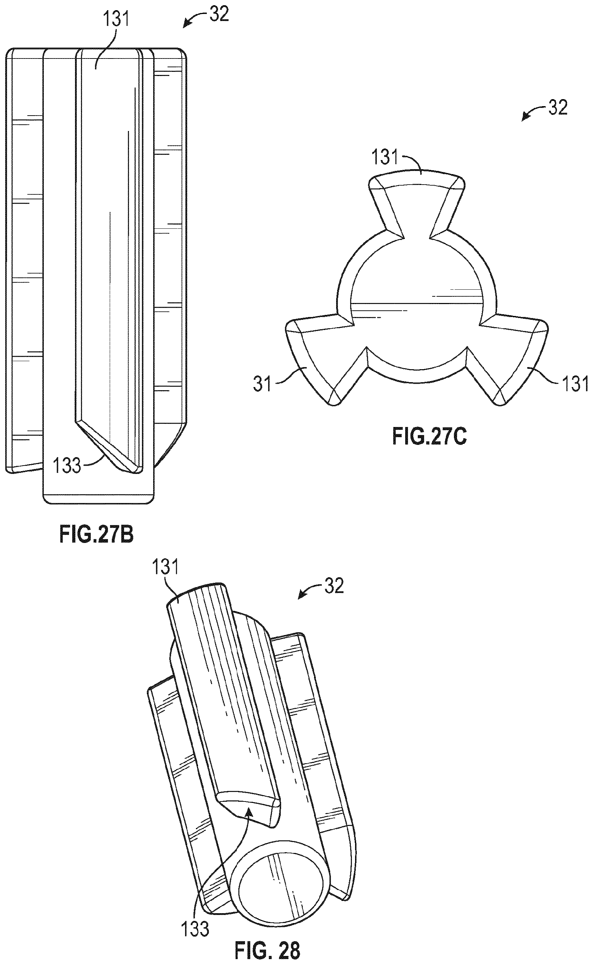

The rotary latch pusher is shown in detail in Figs. 27A-28. The pusher 32 has a plurality of fins 131 that protrude radially outwardly from the center of the pusher. The fins 131 also extend down a majority of the height of the pusher 32. In the embodiment shown in Figs. 27A-27C, the pusher has three fins. It should be appreciated that, in other embodiments, any suitable number of fins may be used, such as 2, 4, 5 or 6 fins. The sloped surface 133 at the bottom of the fins is visible in the side view shown in Fig. 27B and in the bottom perspective view shown in Fig. 28. Furthermore, as seen in the top down view of Fig. 27C, each fin also flares outwardly at the ends. The pusher is rotationally symmetric about a longitudinal axis.

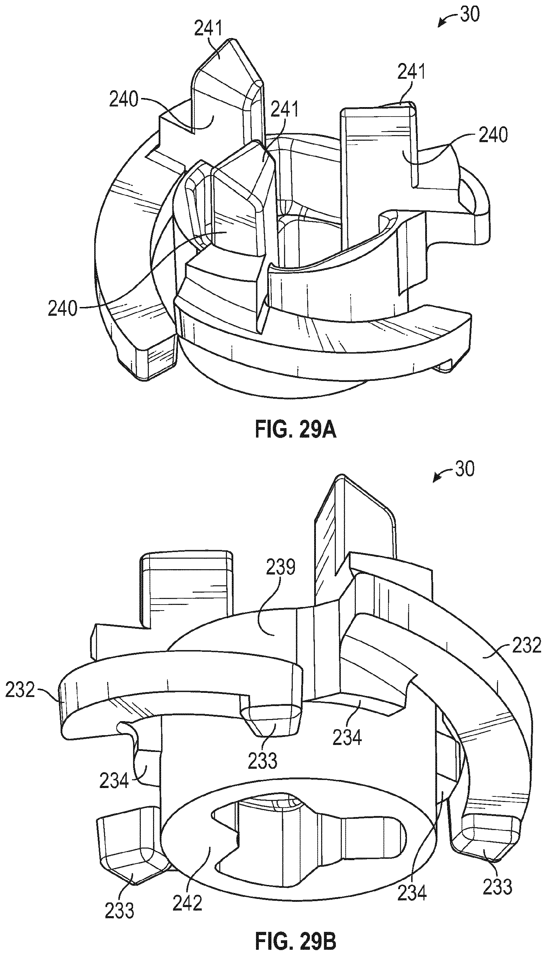

The rotary latch is shown in detail in Figs. 29A-29C. As seen in Fig. 29A, the rotary latch 30 has a plurality of legs 240, each having a sloped surface 241. The legs 240 are arranged in a circular configuration about a longitudinal axis. As seen in Fig. 29B, the rotary latch 30 also has a plurality of arms 232, each having ends 233. The arms are cantilevered from the main body 239 of the rotary latch 30. The arms are shaped and made of a material such that the ends 233 deflect when the arm is subjected to a sufficient amount of force. As a result, the arms 232 behave like springs, each having a stiffness, also referred to as its k-value, or spring constant. In some embodiments, the combined stiffness of all of the arms is greater than the stiffness of the retraction actuator.

As best seen in the bottom perspective views shown in Figs. 29B and 29C, the rotary latch 30 also includes protruding surfaces 234. It is these protruding surfaces 234 that rest upon the ledge surfaces 55 of the carrier protrusions 51 prior to actuation of the device. In some cases, one reason for such an arrangement is to prevent contact between the rotary latch arms 232 and the carrier 50 prior to actuation of the device. This avoids putting stress on the arms 232 prior to actuation. In some cases, prolonged stress on the arms may fatigue the arms and lead to inelastic deformation, cracking or other structural failure. When the protruding surfaces 234 of the rotary latch 30 are resting upon the ledge surfaces 55 of the carrier protrusions, 51 the side surfaces 53 of the protrusions 51 may physically obstruct the rotary latch 30 from rotating in one direction. In the embodiment shown in Fig. 26, the side surfaces 53 would prevent the rotary latch from rotating counter clockwise while the protruding surfaces 234 of the rotary latch rest upon the ledge surfaces 55.

During actuation, the rotary latch 30 rotates off of these protrusions 51. Once the rotary latch 30 has cleared the ledge surfaces of the protrusions, the arm ends 233 come into contact with the top surface of the carrier 50. The bottom surface 242 of the main body 239 of the rotary latch 30 is the surface that contacts the deployment actuator to "trip" and actuate the deployment actuator. Actuation of the deployment actuator is delayed until after the carrier 50 bottoms out against the base 100 due to contact between the arms 232 and the top surface of the carrier 50. In some cases, one reason for delaying actuation of the deployment actuator until after the carrier 50 bottoms out against the base 100 is to ensure that the flow activator is at a proper distance relative to the subj ect's skin prior to deployment of the flow activator. If the flow activator is deployed prior to the carrier 50 bottoming out against the base or otherwise reaching a proper location, the flow activator may be unable to reach the subject's skin when deployed.

Initial contact between the arms 232 and carrier 50 prevents the bottom surface 242 of the rotary latch from exerting force against the deployment actuator by keeping the bottom surface 242 spaced from the deployment actuator. When the arms 232 begin to deflect, the bottom surface 242 is then in sufficient contact with the deployment actuator to exert a force on the deployment actuator and actuate the deployment actuator. Complete deflection of the arms occurs after the carrier 50 bottoms out against the base because, as discussed previously, the collective stiffness of the arms is greater than the stiffness of the retraction actuator, which is sandwiched between the carrier 50 and the base.

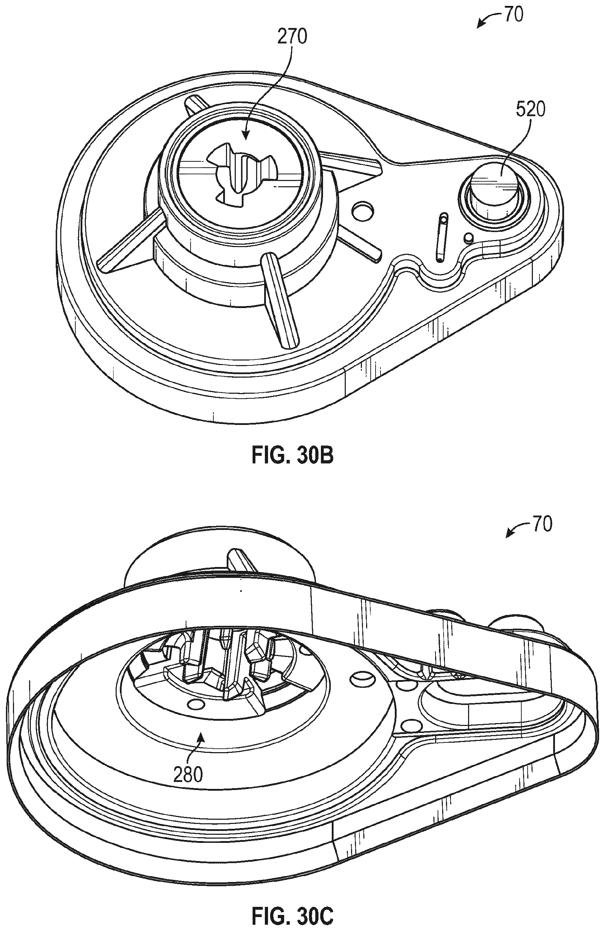

Details of the cap body, which interacts with the rotary latch pusher and the rotary latch, will now be described. The cap body is shown in detail in Figs. 30A-30G. As seen in the top down view shown in Fig. 30A and the top perspective view shown in Fig. 30B, the cap body 70 includes a passage 270. As seen in Fig. 30A, the outer shape of the passage 270 matches the outer shape of the rotary latch pusher 32 such that the pusher can slide through the passage 270. In some embodiments, the passage 270 includes lobe cutouts 271 that match the shape of the fins 131 of the pusher 32.

In some embodiments, the cap body is also connected to the indicator 520. The cap body may be integrally formed with the cap body so that the indicator and cap body are a single, monolithic piece. For example, the cap body and indicator may be a single molded piece. In one embodiment, the cap body and indicator is molded from a transparent or translucent material. In other embodiments, the indicator is a separate component that is attached to the cap body.

On the underside of the cap body, as seen in the bottom perspective views shown in Figs. 30C-30E, the cap body includes an interface 280 that interacts with the rotary latch pusher and the rotary latch. Detailed views of the interface are also shown in Figs. 30F-30G, which are close-up views of the underside of the cap body. As best seen in the detailed views of Fig. 30E-30G, the interface includes a plurality of indentations 286. In some embodiments, the indentations are V-shaped. Prior to actuation of the device, the tops of the legs 240 (see FIG. 29A) of the rotary latch 30 are positioned within these indentations, with each leg received within an indentation. The interface 280 also includes a group of two types of protrusions that alternate in sequence. The first protrusion type 281 has a sloped surface 283 that interacts with the sloped surfaces 241 at the tops of the rotary latch legs 240. In front of each indentation 286 is a channel formed between a pair of protrusion type one 281 and protrusion type two 282. These channels may receive at least a portion of the legs 240 of the rotary latch 30. As the rotary latch 30 moves downward, at least a portion of the legs slide within these channels.

When the rotary latch pusher 32 pushes down on the rotary latch 30, the rotary latch 30 initially moves downward. As a result, the legs 240 of the rotary latch 30 move out of the indentations 286 of the cap body 70. As the rotary latch 30 moves downward, at least a portion of the legs slide within the channels positioned in front of the indentations. Once the rotary latch 30 moves down to a point where the sloped surfaces on the legs of the rotary latch are at the level of the sloped surfaces 283 on the protrusions 281 of the cap body, because the rotary latch is urged to rotate due to its interaction with the rotary latch pusher, the sloped surfaces on the rotary latch contact and slide against the sloped surfaces 283 on the cap body 70. The angle on the sloped surfaces 283 of the cap body 70 urge the legs of the rotary latch up into grooves 287 (best seen in FIGS. 30F-30G) formed between adjacent protrusions. The grooves 287 are recessed at a higher height than the indentations 286, thus allowing the rotary latch to rise, during retraction, to a higher height than its initial pre-actuation position.

Prior to device actuation, the rotary latch is at a first position. After device actuation, the rotary latch moves down in a deployment direction toward a second position as the legs of the rotary latch slide through the channels in front of the indentations 286. Once the top of the legs of the rotary latch clear the protrusions, the rotary latch is urged to rotate. With the force from the retraction actuator pushing the rotary latch upward, the rotary latch is urged up into the grooves 287 in a retraction direction, ending at a final, third position that is higher than the first position. In other words, the pre-actuation position of the rotary latch is closer than its post-actuation position to the device opening.

According to one aspect, the device includes features that assist in moving fluid from the device opening to the storage chamber. Features located at the bottom of the device will now be described in detail.

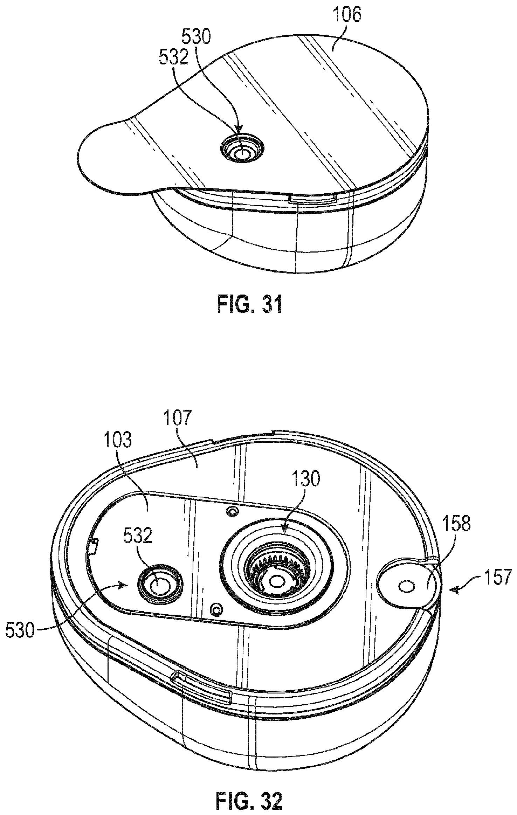

As seen in Fig. 31 and as previously described, the bottom of the device includes an interface cover106. In some embodiments, the bottom of the device also includes a blood access port 530 closed off by a seal 532. The blood access port 530 connects to the storage chamber inside the device. Fluid stored inside the storage chamber can be removed via the blood access port 530.

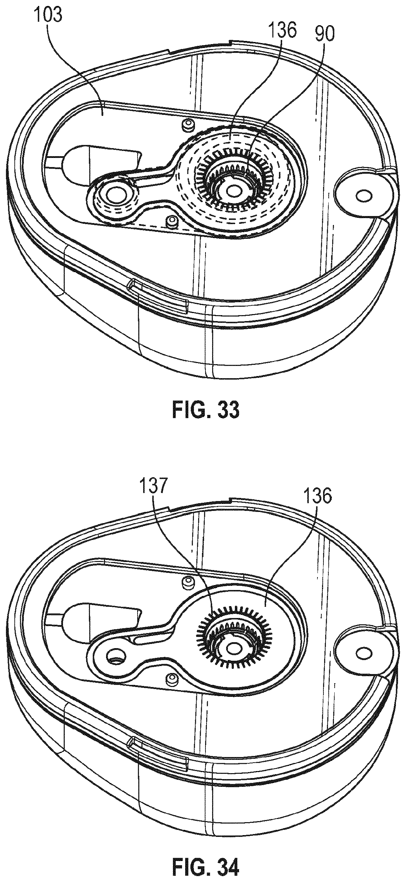

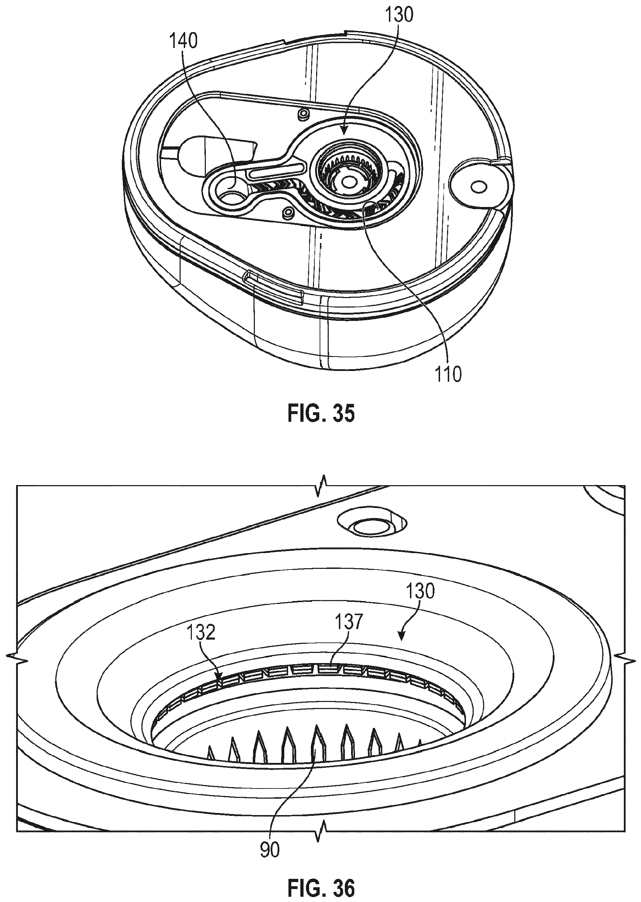

Moving below the release liner, as seen in Fig. 32, in which the release liner and interface adhesive are hidden from view, the base of the device includes a base plate 103. In some embodiments, the base plate is a component that is formed separately from the main base body 107. In some embodiments, as seen in Fig. 33, in which the base plate 103 is shown in phantom, and Fig. 34, in which the base plate is hidden from view, a covering element 136 is sandwiched between the main base body and the base plate 103. Having the base plate 103 being formed as a separate component from the main base body may facilitate insertion of the covering element 136 into the device during manufacturing of the device. For example, with the base plate 103 removed from the main base body, the covering element is inserted in place. The base plate 103 is then placed over the covering element and attached to the main base body. Turning to Fig. 35, in which the covering element is hidden from view, a channel 110 connecting the device opening 130 to the storage chamber 140 can be seen.

According to one aspect, the inventors have recognized the need for a controlled release of anticoagulant into blood flowing into the device.

The inventors have recognized that, when blood flows across a material that is coated with anticoagulant, a large amount of the anticoagulant may be carried off with the initial volume of fluid that flows over the anticoagulant. This may leave an insufficient amount of anticoagulant left for the remaining fluid that is to enter the device, which could result in clotting of the remaining fluid. The inventors have appreciated the need to create a device that has a controlled release of anticoagulant into the incoming fluid such that there is sufficient anticoagulant for remaining fluid after an initial volume of fluid has entered the device.

In some embodiments, the controlled release of anticoagulant is accomplished using a covering member that limits the exposure of the anticoagulant to incoming fluid to limit the rate of dissolution into the incoming fluid. In some embodiments, the covering my include cutouts. In some cases, these cutouts define extending fingers.

A seen in Fig. 36, the opening 130 of the device includes a capillary ring 132. The fingers 137 of the covering element 136 overlap with the surfaces of the capillary ring in a radially inward direction.

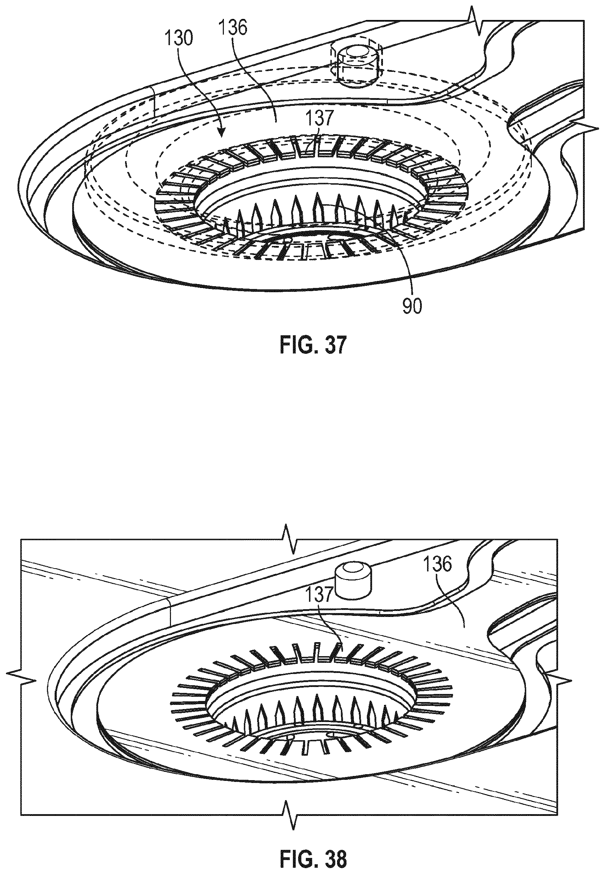

The covering element 136 is best seen in Fig. 37, in which the base plate 103 is shown in phantom, and Fig. 38, in which the base plate is hidden from view. The covering element 136 includes a plurality of fingers 137. The fingers are directed radially inwardly and have slits separating adjacent fingers.

The device may include an anticoagulant or a stabilizing agent for stabilizing the fluid withdrawn from the skin and/or beneath the skin. As a specific non-limiting example, an anticoagulant may be used for blood withdrawn from the skin. Examples of anticoagulants include, but are not limited to, heparin, citrate, thrombin, oxalate, ethylenediaminetetraacetic acid (EDTA), sodium polyanethol sulfonate, acid citrate dextrose. Other agents may be used in conjunction with or instead of anticoagulants, for example, reagents, stabilizing agents such as solvents, diluents, buffers, chelating agents, enzyme inhibitors (ie. Protease or Nuclease inhibitor), antioxidants, binding agents, preservatives, antimicrobials, or the like. Examples of preservatives include, for example, benzalkonium chloride, chlorobutanol, parabens, or thimerosal. Non-limiting examples of antioxidants include ascorbic acid, glutathione, lipoic acid, uric acid, carotenes, alpha-tocopherol, ubiquinol, or enzymes such as catalase, superoxide dismutase, or peroxidases. Examples of microbials include, but are not limited to, ethanol or isopropyl alcohol, azides, or the like. Examples of chelating agents include, but are not limited to, ethylene glycol tetraacetic acid or ethylenediaminetetraacetic acid. Examples of buffers include phosphate buffers such as those known to ordinary skill in the art.

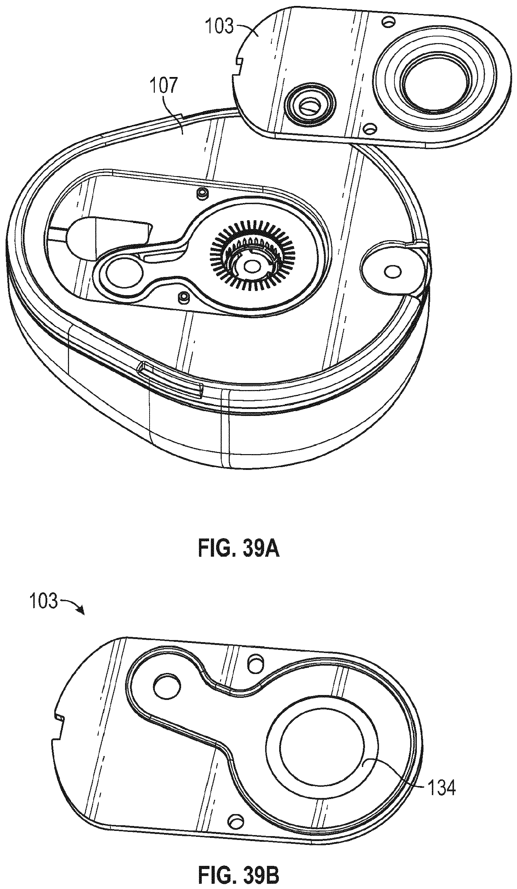

In some embodiments, the capillary ring 132 is coated with an anticoagulant. In the embodiment shown in Fig. 39A, the capillary ring 132 is formed from the combination of the base plate 103 and the main base body 107, where the base plate 103 defines one surface of the capillary ring 132 and the main base body 107 defines a second opposing surface of the capillary ring. The external surface of the base plate 103 is shown in Fig. 39A. In Fig. 39B, the base plate 103 has been flipped around to show the inside surface of the base plate. The inside surface may have a surface 134 that defines one surface of the capillary ring 132. In some embodiments, the inside surface of the base plate is coated with an anticoagulant such that a portion of the capillary ring is coated with anticoagulant. The coating may be limited to the surface 134 defining one surface of the capillary ring or may extend beyond the surface. The covering element is positioned on the capillary ring such that the fingers 137 overlap with the anticoagulant-coated surface of the capillary ring. Without wishing to be bound by theory, these fingers may aid in controlled release of anticoagulant into the fluid flowing into the device. Due to the presence of the covering element, physical contact between incoming fluid flow and anticoagulant occurs only at the spaces between the fingers. The presence of these fingers limits the exposure of the anticoagulant coating to incoming fluid and thereby limits the rate of dissolution of anticoagulant into the incoming fluid. The number and size of the spaces between the fingers may help to control the rate of dissolution of anticoagulant into the incoming fluid.

It should be appreciated that the covering element may have any suitable number of fingers to help provide a suitable anticoagulant dissolution rate. In some embodiments, the covering may include 1 to 40 fingers, at least 40 fingers, 40 to 60 fingers, or any other suitable number of fingers. In some cases, the number of fingers corresponds with the number of spaces between the fingers. In some embodiments, the covering element includes no fingers at all In some embodiments, the covering element has other types of spaces such as windowed cut-outs. In some embodiments, the device does not include a covering element at all.

The covering element may comprise a thin, flexible film or layer made from a plastic or a metal. In some embodiments, the covering element is a MYLAR sheet coated with a hydrophilic adhesive. For example, in some embodiments, the covering element is ARFLOW 92804 from ADHESIVES RESEARCH. The covering element may be attached to the base by any suitable means, for example, via an adhesive.

In some embodiments, the fingers may be hydrophilic to help to wick fluid into the capillary ring. The fingers may be made from a hydrophilic material and/or maybe be coated with a hydrophilic material. In some cases, the hydrophilic and geometric properties of the fingers may serve to disrupt beading of the fluid.

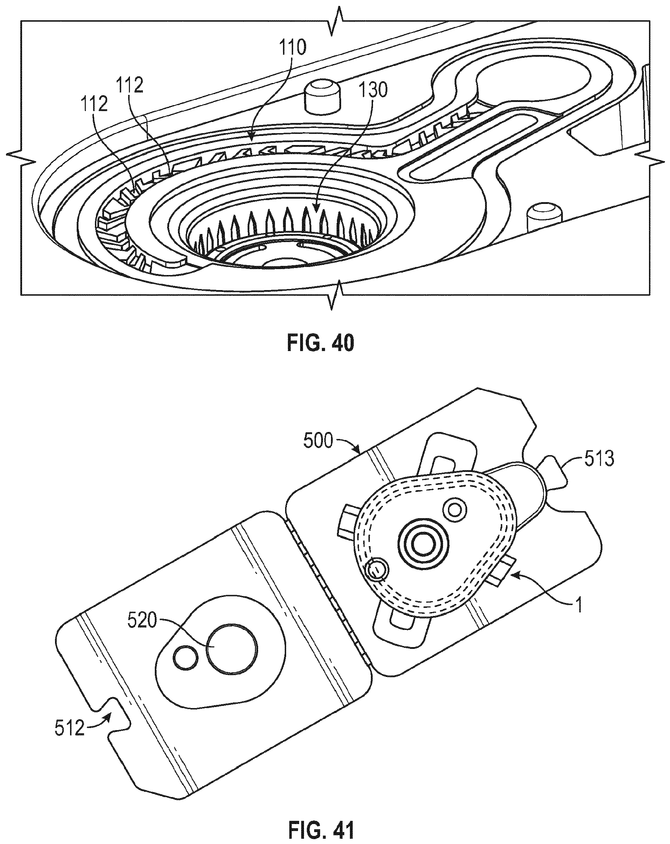

As seen in Fig. 40, the channel 110 includes a curved path and begins from the side of the opening furthest from the storage chamber. According to one aspect, the inventors have recognized the ability to use a channel to facilitate mixing between incoming fluid and an added substance, such as an anticoagulant. In some cases, when the device is used to collect blood, the blood may begin to clot. Clotting of the blood may block the inlet channel into the storage chamber, preventing a desired volume of blood to be collected. In some cases, clotting of the blood may also affect the usefulness of the blood as a specimen for testing. As such, the inventors have recognized the need for an arrangement in which incoming blood does not clot as it enters the device.

In some embodiments, the channel may include indentations 112. In some embodiments, these indentations are chevron-shaped. It should be appreciated, however, that other shapes are possible, for example, rectangular, triangular, rhombus-shaped, or any other suitable shape. In some cases, these indentations may help to mix the flow from side to side, which may help to promote mixing between the fluid and anticoagulant. In some embodiments, flow remains laminar as fluid travels through the channel 110.

In some cases, the channel 110 may be designed with a tortuous pathway or other geometries that elongate the pathway to give the fluid an increased opportunity for mixing and/or to provide greater surface area for anticoagulant coating, or coating of another substance suitable for mixing with the fluid, such as a reagent.

In some embodiments, the channel 110 may be coated with an anticoagulant.

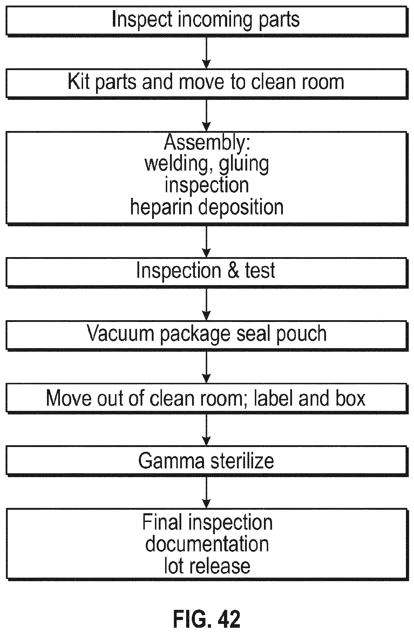

The various components of the device are assembled together and heparin or another anticoagulant is deposited in desired areas of the device. In some embodiments, the components of the device are assembled in a vacuum chamber under vacuum and then sealed to maintain vacuum pressure inside the device. In other embodiments, vacuum may be introduced into the vacuum chamber by evacuating air out of the vacuum chamber. In some embodiments, the assembled device is held in a rigid covering that is shaped to receive the device. In the illustrative embodiment shown in Fig. 41, the covering 500 is a clam-shell and may include a recessed portion 520 that is shaped to receive and cover the device actuator. The recessed portion may be made from a rigid material to prevent inadvertent actuation of the device. In some embodiments the covering is made of plastic. The clam-shell covering 500 may include closing elements that hold the clam-shell closed. For example, the closing elements may include a protrusion 513 on one side and an indentation 512 on the other side, the protrusion be shaped to fit within the indentation 512 to hold the covering closed. The protrusion and indentation may both have a tapered shape. The device (which may be, in some embodiments, held within a rigid covering) may be placed within a vacuum packaging and sealed. The device undergoes a sterilization process, for example, gamma sterilization. One illustrative embodiment of a manufacturing process is shown in Fig. 42.

As described above, an interface cover such as a release liner may be used to prevent the interface (e.g., a hydrogel adhesive) from prematurely adhering to other objects. It should be appreciated that other types of interface covers other than a release liner may be used. In some embodiments, the interface cover is a tray that blocks the interface from contacting other objects. In some embodiments, the tray may be removably attached to the device. When the device is ready to be used, a user may remove the tray to uncover the interface. In order to uncover the interface, the tray may be completely detached from the device, or may remain attached to the device but in an uncovered position (e.g., pivoted away to uncover the interface). In some embodiments, when the tray is attached to the device and in the covered position, the tray may be spaced from the interface such that no portion of the interface is in contact with the tray. In some embodiments, the tray may have an inner surface that faces the interface but is spaced away from the interface. In some embodiments, the inner surface may be recessed away from the interface. In some embodiments, having the interface out of contact with the tray may help to preserve the shape and function of the interface. To protect the interface, the tray may be load-bearing and, due to the tray being out of contact with the interface, may avoid transferring the load that the tray experiences to the interface. In embodiments where the interface may be made of a material that is vulnerable to shape or functionality changes when bearing a load, e.g., a hydrogel that can be squeezed out of place, out of shape, or lose functionality due to being squeezed, the tray may help to protect the interface from being subjected to potentially detrimental forces prior to using the device.

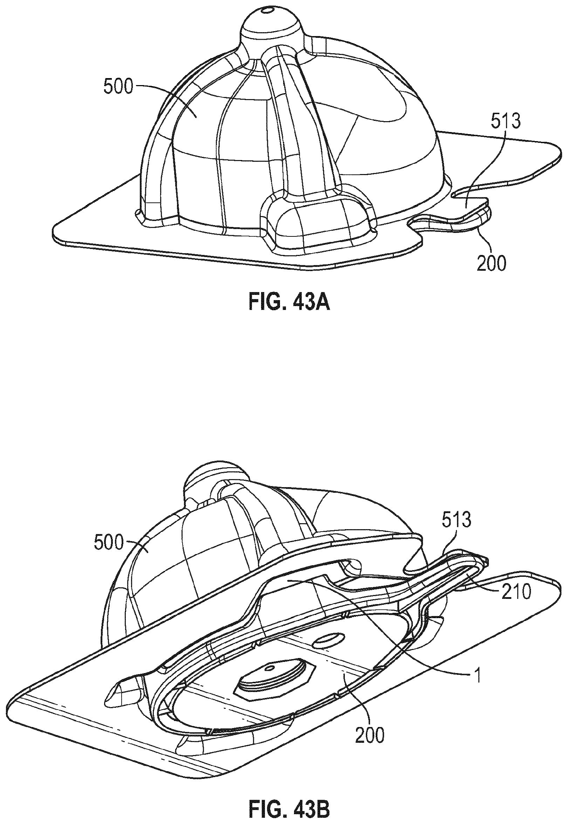

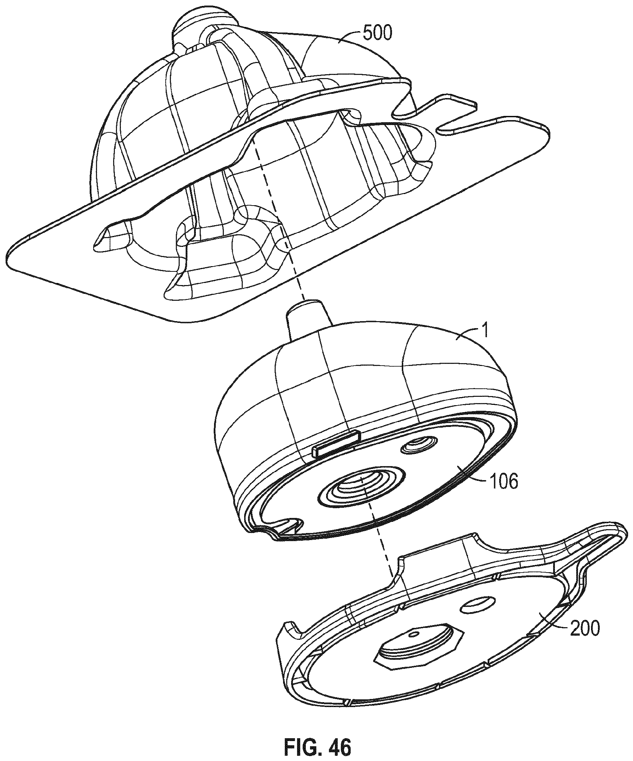

One illustrative embodiment of an interface cover is shown in FIGS. 43A-46, which depict a device 1 within a covering 500 and an interface cover 200 covering an interface 106 (best seen in FIG. 46) at the bottom of the device. In some embodiments, such as those seen in FIGS. 43A-46, the interface cover is a protective tray that attaches to the device 1 and covers over the interface of the device to prevent contact of the interface with other objects prior to use.

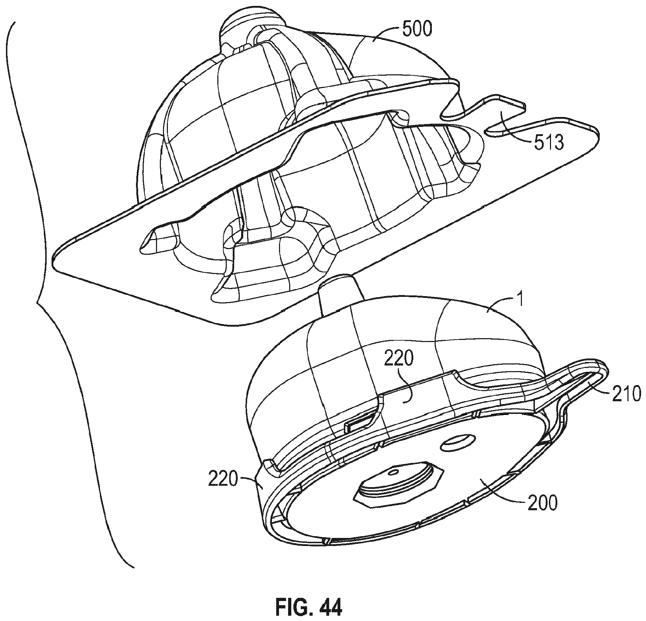

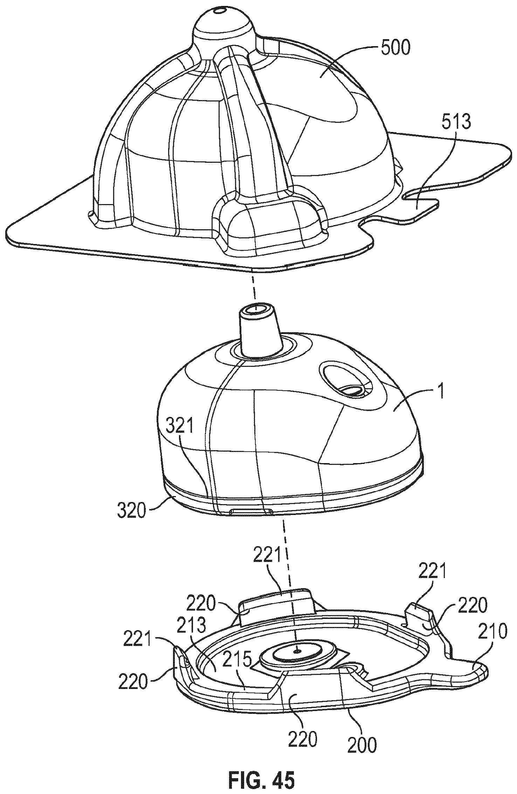

The tray 200 may snap onto the bottom of the device 1 by engagement between engaging features on the tray that correspond with engaging features on the device 1. As best seen in FIGS. 44 and 45, the tray 200 includes engagement fingers 220 that have outwardly protruding prongs 221 that are positioned and shaped to latch onto a protruding ledge 320 on the device 1. The protruding ledge 320 may be on a side of the device 1. The protruding ledge 320 may have a top surface 321 and may run continuously along the side if the device 1, or may be in the form of shortened segments along the side of the device, spaced and positioned to align with the fingers 220 of the tray 200. The fingers 220 and ledge 320 may be reversibly attached and detached from one another. To attach the tray to the device, the device and tray may be pressed into contact with one another until the fingers 220 flex outwardly to receive the ledge 320, and the fingers 220 snap back into place with the prongs 221 of the fingers resting on or above the top surface 321 of the ledge 320. The prongs 221 may serve to retain attachment between the tray 200 and the device 1. To detach the tray from the device, a user may pull the tray and device away from one another until the fingers 220 flex outwardly to release the ledge 320. In some embodiments, the tray 200 may include a protruding tab 210. A user may pull on the tab 210 in a direction away from the device 1 to detach the tray 200 from the device.

It should be understood that, in some embodiments, the position of the fingers and ledge may be reversed such that the fingers are on the device and the ledge(s) is/are on the tray. It should also be understood that any suitable number of fingers and/or ledges may be used. The embodiment shown the figures includes four fingers and one continuous ledge, but other combinations are possible. For example, in some embodiments, 1, 2, 3, 5, 6, 7, 8, 9, 10 or any other suitable number of fingers and/or ledges may be used.

In some embodiments, as seen in FIG. 45, the tray 200 includes an inner surface 213 that faces towards the interface 106 of the device when the tray is attached to the device and in a covering position. In some embodiments, the height of the fingers 220 holds the tray in a position such that the inner surface 213 is spaced from the interface, out of contact with the interface. In some embodiments, the tray includes a rim 215 from which the fingers 220 protrude, and the inner surface 213 is recessed relative to the rim such that the inner surface 213 and the surface of the rim 215 are on different planes.

In some embodiments, as seen in FIGS. 43A and 43B, the covering 500 may include a protrusion 513, and the tab 210 of the interface cover 200 may align with the protrusion 513 of the covering 500 when the device 1 is fully received into the covering 500.

Further details regarding optional arrangements for needles, which may be included as part of a flow activator, are provided below.

As mentioned above, needles included with a flow activator may be arranged in a variety of different ways, depending on the intended application. For example, the needle(s) may have a length of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, less than about 10 micrometers, etc. The needle(s) may also have a largest cross-sectional dimension of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, less than about 10 micrometers, etc. For example, in one embodiment, the needle(s) may have a rectangular cross section having dimensions of 175 micrometers by 50 micrometers. In one set of embodiments, the needle(s) may have an aspect ratio of length to largest cross-sectional dimension of at least about 2:1, at least about 3:1, at least about 4:1, at least 5:1, at least about 7:1, at least about 10:1, at least about 15:1, at least about 20:1, at least about 25:1, at least about 30:1, etc

In one embodiment, the needle(s) is(are) a microneedle(s). Typically, a microneedle will have an average cross-sectional dimension (e.g., diameter) of less than about a millimeter. It should be understood that references to "needle" or "microneedle" as discussed herein are by way of example and ease of presentation only, and that in other embodiments, more than one needle and/or microneedle may be present in any of the descriptions herein. As an example, microneedles such as those disclosed in U.S. Patent No. 6,334,856, issued January 1, 2002, entitled "Microneedle Devices and Methods of Manufacture and Use Thereof," by Allen, et al., may be used to deliver to and/or withdraw fluids (or other materials) from a subject. The microneedles may be hollow or solid, and may be formed from any suitable material, e.g., metals, ceramics, semiconductors, organics, polymers, and/or composites. Examples include, but are not limited to, medical grade stainless steel, titanium, nickel, iron, gold, tin, chromium, copper, alloys of these or other metals, silicon, silicon dioxide, and polymers, including polymers of hydroxy acids such as lactic acid and glycolic acid polylactide, polyglycolide, polylactide-co-glycolide, and copolymers with polyethylene glycol, polyanhydrides, polyorthoesters, polyurethanes, polybutyric acid, polyvaleric acid, polylactide-co-caprolactone, polycarbonate, polymethacrylic acid, polyethylenevinyl acetate, polytetrafluorethylene, polymethyl methacrylate, polyacrylic acid, or polyesters.

In some cases, more than one needle or microneedle may be used. For example, arrays of needles or microneedles may be used, and the needles or microneedles may be arranged in the array in any suitable configuration, e.g., periodic, random, etc. In some cases, the array may have 3 or more, 4 or more, 5 or more, 6 or more, 10 or more, 15 or more, 20 or more, 35 or more, 50 or more, 100 or more, or any other suitable number of needles or microneedles. Typically, a microneedle will have an average cross-sectional dimension (e.g., diameter) of less than about a micron.

Those of ordinary skill in the art can arrange needles relative to the skin or other surface for these purposes including, in one embodiment, introducing needles into the skin at an angle, relative to the skin's surface, other than 90°, i.e., to introduce a needle or needles into the skin in a slanting fashion so as to limit the depth of penetration. In another embodiment, however, the needles may enter the skin or other surface at approximately 90°.

In some cases, the needles (or microneedles) may be present in an array selected such that the density of needles within the array is between about 0.5 needles/mm² and about 10 needles/mm2, and in some cases, the density may be between about 0.6 needles/mm² and about 5 needles/mm², between about 0.8 needles/mm² and about 3 needles/mm², between about 1 needles/mm² and about 2.5 needles/mm², or the like. In some cases, the needles may be positioned within the array such that no two needles are closer than about 1 mm, about 0.9 mm, about 0.8 mm, about 0.7 mm, about 0.6 mm, about 0.5 mm, about 0.4 mm, about 0.3 mm, about 0.2 mm, about 0.1 mm, about 0.05 mm, about 0.03 mm, about 0.01 mm, etc.

In another set of embodiments, the needles (or microneedles) may be chosen such that the area of the needles (determined by determining the area of penetration or perforation on the surface of the skin of the subject by the needles) allows for adequate flow of fluid to or from the skin and/or beneath the skin of the subject. The needles may be chosen to have smaller or larger areas (or smaller or large diameters), so long as the area of contact for the needles to the skin is sufficient to allow adequate blood flow from the skin of the subject to the device. For example, in certain embodiments, the needles may be selected to have a combined skin-penetration area of at least about 500 nm², at least about 1,000 nm², at least about 3,000 nm², at least about 10,000 nm², at least about 30,000 nm², at least about 100,000 nm², at least about 300,000 nm², at least about 1 microns², at least about 3 microns², at least about 10 microns², at least about 30 microns², at least about 100 microns², at least about 300 microns², at least about 500 microns², at least about 1,000 microns², at least about 2,000 microns², at least about 2,500 microns², at least about 3,000 microns², at least about 5,000 microns², at least about 8,000 microns², at least about 10,000 microns², at least about 35,000 microns², at least about 100,000 microns², at least about 300,000 microns², at least about 500,000 microns², at least about 800,000 microns², at least about 8,000,000 microns², etc., depending on the application.

The needles or microneedles may have any suitable length, and the length may be, in some cases, dependent on the application. For example, needles designed to only penetrate the epidermis may be shorter than needles designed to also penetrate the dermis, or to extend beneath the dermis or the skin. In certain embodiments, the needles or microneedles may have a maximum penetration into the skin of no more than about 3 mm, no more than about 2 mm, no more than about 1.75 mm, no more than about 1.5 mm, no more than about 1.25 mm, no more than about 1 mm, no more than about 900 microns, no more than about 800 microns, no more than about 750 microns, no more than about 600 microns, no more than about 500 microns, no more than about 400 microns, no more than about 300 microns, no more than about 200 microns, no more than about 175 micrometers, no more than about 150 micrometers, no more than about 125 micrometers, no more than about 100 micrometers, no more than about 75 micrometers, no more than about 50 micrometers, etc. In certain embodiments, the needles or microneedles may be selected so as to have a maximum penetration into the skin of at least about 50 micrometers, at least about 100 micrometers, at least about 300 micrometers, at least about 500 micrometers, at least about 1 mm, at least about 2 mm, at least about 3 mm, etc.

In one set of embodiments, the needles (or microneedles) may be coated. For example, the needles may be coated with a substance that is delivered when the needles are inserted into the skin. For instance, the coating may comprise heparin, an anticoagulant, an anti-inflammatory compound, an analgesic, an anti-histamine compound, etc to assist with the flow of blood from the skin of the subject, or the coating may comprise a drug or other therapeutic agent such as those described herein. The drug or other therapeutic agent may be one used for localized delivery (e.g., of or proximate the region to which the coated needles or microneedles are applied), and/or the drug or other therapeutic agent may be one intended for systemic delivery within the subject.

While aspects of the invention have been described with reference to various illustrative embodiments, such aspects are not limited to the embodiments described. Thus, it is evident that many alternatives, modifications, and variations of the embodiments described will be apparent to those skilled in the art. Accordingly, embodiments as set forth herein are intended to be illustrative, not limiting. Various changes may be made without departing from the spirit of aspects of the invention.

Preferred embodiments of the invention are set out in the following paragraphs.

According to a first aspect of the invention, there is provided a device for receiving fluid from a subject, comprising:
a housing including an opening to receive fluid into the housing;
a device actuator;
one or more needles being arranged to cause fluid to be released from the subject;
a deployment actuator that moves the one or more needles in a deployment direction toward a subject; and
a rotatable element that contacts the deployment actuator to actuate the deployment actuator after actuation of the device actuator, the rotatable element being rotatable relative to the housing,
wherein actuation of the device actuator causes the rotatable element to rotate and move toward the deployment actuator.

In embodiments of the first aspect of the invention, the device may further comprise a retraction actuator that moves the flow activator in a retraction direction, wherein the rotatable element may prohibit the retraction actuator from moving the one or more needles in the retraction direction prior to actuation of the device actuator and rotates relative to the housing to permit the retraction actuator to move the one or more needles in the retraction direction.

In embodiments of the first aspect of the invention, the rotatable element may have a pre-actuation position and a post-actuation position, the pre-actuation position being closer than the post-actuation position to the device opening.

In embodiments of the first aspect of the invention, the device may further comprise a pusher element that interacts with the device actuator and the rotatable element, wherein actuation of the device actuator may cause the pusher to move downward and exert a force on the rotatable element, causing the rotatable element to move downward and to rotate.

In embodiments of the first aspect of the invention, the rotatable element may be linearly movable relative to the housing.

In embodiments of the first aspect of the invention, the device may further comprise a carrier coupled to the deployment actuator.

In embodiments of the first aspect of the invention comprising the carrier, the device may further comprise at least one spring between the rotatable element and the carrier. The at least one spring may be integrally formed with the rotatable element. The spring may comprise an arm cantilevered from the rotatable element. The arm may be curved or slanted.

In embodiments of the first aspect of the invention comprising the carrier, the device may further comprise a plurality of springs between the rotatable element and the carrier.

In embodiments of the first aspect of the invention, prior to device actuation, the rotatable element may be spaced from the deployment actuator.

In embodiments of the first aspect of the invention, after retraction of the deployment actuator, the rotatable element may be spaced from the deployment actuator.

In embodiments of the first aspect of the invention, the rotatable element may move linearly relative to the deployment actuator.

In embodiments of the first aspect of the invention, the rotatable element may rotate from a pre-actuation position to a post-actuation position, the device being locked from further actuation with the rotatable element in the post-actuation position. In such embodiments, the rotatable element may be locked from further rotation in the post-actuation position.

In embodiments of the first aspect of the invention, the device may further comprise a pusher that contacts the rotatable element to rotate the rotatable element. In such embodiments, prior to actuation, the pusher may be positioned between the rotatable element and the device actuator. In other such embodiments, during actuation, the pusher may be in contact with the device actuator. Preferably, after actuation, the pusher becomes spaced from the device actuator.

In embodiments of the first aspect of the invention comprising the pusher, as the rotatable element rotates during actuation, the pusher may move through a hole in the rotatable element.

In embodiments of the first aspect of the invention comprising the pusher, the pusher may move linearly through the hole in the rotatable element.

In embodiments of the first aspect of the invention, the device may further comprise a device interface that provides a seal against skin. Preferably the interface comprises a hydrogel. More preferably the device further comprises an interface covering that covers the device interface. Even more preferably the interface covering is spaced from contact with the device interface.

According to a second aspect of the invention, there is provided a device for receiving fluid from a subject, comprising:
a housing comprising a base;
a deployment actuator;
a carrier coupled to the deployment actuator;
a deployment actuator initiating element that actuates the deployment actuator by contacting and exerting a force on the deployment actuator, the initiating element having at least one spring member and the initiating element being positioned on the carrier;
a retraction actuator positioned between the carrier and the base such that movement of the carrier towards the base causes the retraction actuator to compress;
wherein a stiffness of the at least one spring member is greater than a stiffness of the retraction actuator.

In embodiments of the second aspect of the invention, the deployment actuator may comprise a bi-stable element.

In embodiments of the second aspect of the invention, the deployment actuator may comprise a snap dome.

In embodiments of the second aspect of the invention, each of the spring members may be an arm that is cantilevered from a main body of the initiating element.

In embodiments of the second aspect of the invention, the initiating element may comprise a rotatable element that rotates relative to the carrier.

In embodiments of the second aspect of the invention, the device may further comprise a device opening through which fluid is received into the device, wherein the initiating element has a pre-actuation position and a post-retraction position, the pre-actuation position being closer in distance to the device opening than the post-retraction position.

In embodiments of the second aspect of the invention, prior to actuation of the device, the at least one spring member may be in a relaxed state. In such embodiments, the carrier may include at least one ledge and a face below the ledge, wherein prior to actuation of the device, the initiating element may rest upon the at least one ledge such that the at least one spring member is in a relaxed state, and upon actuation, the initiating element may move off of the ledge and the at least one spring member may make contact with the face of the carrier.

In embodiments of the second aspect of the invention, the at least one spring member may contact the carrier prior to the initiating element contacting the deployment actuator.

According to a third aspect of the invention, there is provided a device for receiving fluid from a subject, the device comprising:
a housing comprising a base, the base including a hole and a device opening, the hole being spaced from the device opening;
a deployment actuator;
a flow activator comprising a plurality of needles; and
an intermediate component coupling the flow activator to the deployment actuator, the intermediate component extending across the hole and being movable through the hole,
wherein, when the intermediate component is in the retracted position, the intermediate component seals the hole such that fluid communication through the hole is closed.

In embodiments of the third aspect of the invention, the base further comprising a sealing surface surrounding the hole and the intermediate component includes a flange, wherein the flange abuts against the sealing surface to seal the hole. In such embodiments, the device may further comprise a gasket on the sealing surface.

In embodiments of the third aspect of the invention, the device may further comprise a gasket on the intermediate component.

In embodiments of the third aspect of the invention, the intermediate component may comprise a post. In such embodiments, the post may be heat-staked to the deployment actuator.

According to a fourth aspect of the invention, there is provided a device for receiving fluid from a subject, comprising:
a housing comprising a base;
a deployment actuator that moves the one or more needles in a deployment direction toward a subject; and
a carrier coupled to the deployment actuator, wherein upon actuation, the carrier moves toward the device opening prior to actuation of the deployment actuator.

In embodiments of the fourth aspect of the invention, the device may further comprise a retraction actuator positioned between the carrier and the base, wherein the retraction actuator moves the carrier in a retraction direction.

In embodiments of the fourth aspect of the invention, the deployment actuator may be coupled to an underside of the carrier.

In embodiments of the fourth aspect of the invention, the carrier may include a piercing element.

In embodiments of the fourth aspect of the invention comprising the piercing element, the device may further comprise a seal that closes communication between the vacuum chamber and the device opening prior to actuation of the device, wherein after actuation of the device, the carrier moves relative to the housing and the piercing element pierces the seal. In such embodiments, the device may further comprise a retraction actuator positioned between the carrier and the base, wherein the retraction actuator moves the carrier in a retraction direction to cause the piercing element to pierce the seal. In other such embodiments, piercing the seal may occur after movement of the one or more needles in a deployment direction.

According to a fifth aspect of the invention, there is provided a device for receiving fluid from a subject, comprising:
a housing comprising a base;
a deployment actuator;
a carrier coupled to the deployment actuator;
a deployment actuator initiating element that actuates the deployment actuator by contacting and exerting a force on the deployment actuator, the initiating element having at least one spring member and the initiating element being positioned on the carrier,
wherein the at least one spring member contacts the carrier prior to the initiating element contacting the deployment actuator.

In embodiments of the fifth aspect of the invention, the carrier may include at least one ledge and a face below the ledge, wherein prior to actuation of the device, the initiating element may rest upon the at least one ledge, and upon actuation, the initiating element may move off of the ledge and the at least one spring member may make contact with the face of the carrier.

According to a sixth aspect of the invention, there is provided a device for receiving fluid from a subject, comprising:
a housing including an opening to receive fluid into the housing, the housing comprising a base;
a device actuator;
one or more needles being arranged to cause fluid to be released from the subject;
an anticoagulant coating on the base;
a covering element positioned on the anticoagulant coating, the covering element having spaces that expose the anticoagulant coating.

In embodiments of the sixth aspect of the invention, the covering element may comprise a plurality of fingers that extend radially inwardly toward the opening and the spaces are located between the fingers.

In embodiments of the sixth aspect of the invention, the base may include a capillary ring and the anticoagulant coating may be on the capillary ring.

In embodiments of the sixth aspect of the invention, the base may comprise a main base portion and a base plate, and the covering element may be sandwiched between the main base portion and the base plate. Preferably the anticoagulant coating is on the base plate. Preferably the capillary ring is formed by the main base portion and the base plate.

In embodiments of the sixth aspect of the invention, the covering element may comprise a film.

## Claims

1. A device for receiving fluid from a subject, comprising:
a housing comprising a base having a device opening;
a deployment actuator that moves one or more needles in a deployment direction toward a subject;
a carrier coupled to the deployment actuator, wherein upon actuation, the carrier moves toward the device opening prior to actuation of the deployment actuator; and
a retraction actuator positioned between the carrier and the base, wherein the retraction actuator moves the carrier in a retraction direction.

2. The device of claim 1, wherein movement of the carrier towards the base compresses the retraction actuator.

3. The device of claim 2, wherein when the retraction actuator is permitted to decompress, the retraction actuator pushes the carrier up away from the base in the retraction direction.

4. The device of claim 3, wherein when the retraction actuator is permitted to decompress, the retraction actuator pushes the deployment actuator and the one or more needles up away from the base in the retraction direction.

5. The device of claim 1, wherein the base includes an opening that receives fluid into the device and a storage chamber that collects the fluid.

6. The device of claim 1, wherein a bottom of the device includes a release liner and an interface, wherein the release liner is removable to reveal the interface, wherein the interface has adhesive properties and the release liner is used to prevent the interface from prematurely adhering to other objects.

7. The device of claim 1, wherein the carrier holds the deployment actuator such that movement of the carrier moves the deployment actuator along with the carrier.

8. The device of claim 1, wherein the retraction actuator surrounds the deployment actuator.

9. The device of claim 1, further comprising a post that couples the deployment actuator to the one or more needles.

10. The device of claim 1, wherein the device is entirely mechanical and does not require a power source or software electronics.

11. The device of claim 1, wherein vacuum is not pre-packaged within the device and wherein vacuum is generated during actuation of the device.

12. The device of claim 1, further comprising an interface that aids in fluid collection by providing an airtight seal between the device and the subject's skin that prevents escape of fluid.

13. The device of claim 12, wherein the interface comprises a seal enhancing material.

14. The device of claim 1, further comprising a storage chamber and a channel connecting the device opening to the storage chamber.
